# EUROPÄISCHE PATENTSCHRIFT

(11) **EP 1 345 895 B1**
(45) Veröffentlichungstag und Bekanntmachung des Hinweises auf die Patenterteilung: **27.12.2006**
(21) Anmeldenummer: 01271353.3
(22) Anmeldetag: 11.12.2001
(51) Int. Cl.: C07D 205/08, A61K 31/395, A61P 3/06

(54) **NEUE DIPHENZYLAZETIDINONE, VERFAHREN ZU DEREN HERSTELLUNG, DIESE VERBINDUNGEN ENTHALTENDE ARZNEIMITTEL UND DEREN VERWENDUNG ZUR BEHANDLUNG VON LIPIDSTOFFWECHSELSTÖRUNGEN**
NOVEL 1,2-DIPHENZYLAZETIDINONES, METHOD FOR PRODUCING THE SAME, MEDICAMENTS CONTAINING SAID COMPOUNDS, AND THE USE THEREOF FOR TREATING DISORDERS OF THE LIPID METABOLISM
NOUVELLES AZETIDINONES 1,2-DIPHENYLIQUES, LEUR PROCEDE DE PRODUCTION, MEDICAMENTS RENFERMANT CES COMPOSES ET LEUR UTILISATION DANS LE TRAITEMENT DE TROUBLES DU METABOLISME LIPIDIQUE

(30) Priorität: 21.12.2000 DE 10064398; 26.10.2001 DE 10152981
(43) Veröffentlichungstag der Anmeldung: 24.09.2003
(73) Patentinhaber: Sanofi-Aventis Deutschland GmbH, 65929 Frankfurt am Main (DE)
(72) Erfinder: GLOMBIK, Heiner, 65719 Hofheim (DE); KRAMER, Werner, 55130 Mainz-Laubenheim (DE); FLOHR, Stefanie, 4054 Basel (CH); FRICK, Wendelin, 65510 Hünstetten-Beuerbach (DE); HEUER, Hubert, 55270 Schwabenheim (DE); JAEHNE, Gerhard, 65929 Frankfurt (DE); LINDENSCHMIDT, Andreas, 65812 Bad Soden (DE); SCHAEFER, Hans-Ludwig, 65239 Hochheim (DE)
(86) Internationale Anmeldenummer: PCT/EP2001/014531
(87) Internationale Veröffentlichungsnummer: WO 2002/050027

(56) Entgegenhaltungen:
- WO-A-00/63703
- WO-A-97/16455
- WO-A-97/45406
- US-A- 5 756 470
- VACCARO W D ET AL: "Sugar-substituted 2-azetidinone cholesterol absorption inhibitors: enhanced potency by modification of the sugar" BIOORGANIC & MEDICINAL CHEMISTRY LETTERS, OXFORD, GB, Bd. 8, Nr. 3, 3. Februar 1998 (1998-02-03), Seiten 313-318, XP004136870 ISSN: 0960-894X -& VACCARO WD ET AL: BIOORGANIC & MEDICINAL CHEMISTRY LETTERS, Bd. 8, Nr. 1-6, 6. Januar 1998 (1998-01-06), Seiten 35-40, XP004136618 Oxford, GB
- ZAKS, ALEKSEY ET AL: "Enzymic glucuronidation of a novel cholesterol absorption inhibitor, SCH 58235" APPL. BIOCHEM. BIOTECHNOL. (1998), 73(2-3), 205-214, XP001055417
- M. VAN HEEK, C. FARLEY, D.S. COMPTON: "Comparison of activity of the cholesterol absorption inhibitor SCH58235 and its glucuronide SCH60663." BRITISH JOURNAL OF PHARMACO., Bd. 129, 2000, Seiten 1748-1754, XP001057494

## Beschreibung

Die Erfindung betrifft substituierte Diphenylazetidinoneund deren physiologisch verträgliche Salze.

Es sind bereits Diphenylazetidinone (wie z.B. Ezetimibe) sowie deren Verwendung zur Behandlung von Hyperlipidämie sowie Arteriosklerose und Hypercholesterinämie beschrieben worden [vgl. Drugs of the Future 2000, 25(7):679-685) und US 5,756,470].
WO9716455 und Vaccaro et al., Bioorganic& Medicinal Chemistry Letters Bd. 8, Nr. 3, Seiten 35-40 sowie Seiten 313 bis 318 offenbaren Azetidinonderivate.

Der Erfindung lag die Aufgabe zugrunde, weitere Verbindungen zur Verfügung zu stellen, die eine therapeutisch verwertbare hypolipidämische Wirkung entfalten. Insbesondere bestand die Aufgabe darin, neue Verbindungen zu finden, die gegenüber den im Stand der Technik beschriebenen Verbindungen, sehr gering resorbierbar sind. Unter sehr gering resorbierbar wird eine intestinale Resorption kleiner 10%, bevorzugt kleiner oder gleich 5% verstanden.

Die neuen Verbindungen sollen insbesonders eine geringere Resorption als Ezetimibe auf weisen.
Bei geringerer Resorption zeigen pharmazeutische Wirkstoffe in der Regel deutlich weniger Nebenwirkungen.

Die Erfindung betrifft daher Verbindungen der Formel I, 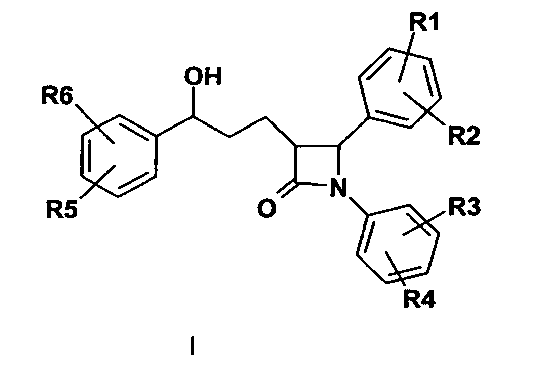 worin bedeuten
- R1, R3: unabhängig voneinander -(CH₂)₀₋₁-NH-(C=O)₀₋₁-(C₀-C₂₅)-Alkylen-(C=O)₀₋₁-N(R7)₀₋₁-LAG ,wobei ein oder mehrere C-Atome des Alkylenrests durch O-Atome ersetzt sein können,
H, F, Cl, Br, I, CF₃, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
SO₂-NH₂, SO₂NH(C₁-C₆)-Alkyl, SO₂N[(C₁-C₆)-Alkyl]₂, S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
- R2, R4, R5, R6 u: nabhängig voneinander -(CH₂)₀₋₁-NH-(C=O)₀₋₁-(C₃-C₂₅)-Alkylen-(C=O)₀₋₁-N(R7)₀₋₁-LAG ,wobei ein oder mehrere C-Atome des Alkylenrests durch O-Atome ersetzt sein können, H, F, Cl, Br, I, CF₃, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
SO₂-NH₂, SO₂NH(C₁-C₆)Alkyl, SO₂N[(C₁-C₆)Alkyl]₂, S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, I, OH, CF₃, NO₂, CN, OCF₃, O-(C₁-C₆)Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
- R7: H, CH₃;
- (LAG): Zuckerrest;
worin einer der Reste R1 oder R3 die Bedeutung -(CH₂)₀₋₁-NH-(C=O)₀₋₁-(C₃-C₂₅)-Alkylen-(C=O)₀₋₁-N(R7)₀₋₁-LAG ,wobei ein oder mehrere C-Atome des Alkylenrests durch O-Atome ersetzt sein können, besitzt, sowie deren pharmazeutisch verträglichen Salze.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Ethansulfon-, Fumar-, Glucon-, Glykol-, Isothion-, Milch-, Lactobion-, Malein-, Apfel-, Methansulfon-, Bernstein-, p-Toluolsulfon-, Wein- und Trifluoressigsäure. Für medizinische Zwecke wird in besonders bevorzugter Weise das Chlorsalz verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nichttherapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze und Solvate wie hierin beschrieben.

Die Verbindungen der Formel I und deren pharmazeutisch verträgliche Salze stellen ideale Arzneimittel zur Behandlung von Lipidstoffwechselstörungen, insbesondere von Hyperlipidämie dar. Die Verbindungen der Formel I eignen sich ebenfalls zur Beeinflussung des Serumcholesterinspiegels sowie zur Prävention und Behandlung arteriosklerotischer Erscheinungen.

Die Verbindung(en) der Formel (I) können auch in Kombination mit weiteren Wirkstoffen verabreicht werden.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,1 mg bis 100 mg (typischerweise von 0,1 mg und 50 mg) pro Tag pro Kilogramm Körpergewicht, z.B. 0,1-10 mg/kg/Tag. Tabletten oder Kapseln, können beispielsweise von 0,01 bis 100 mg, typischerweise von 0,02 bis 50 mg enthalten. Im Falle pharmazeutisch verträglicher Salze beziehen sich die vorgenannten Gewichtsangaben auf das Gewicht des vom Salz abgeleiteten Diphenylazetidinon-lons. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale und perorale (z.B. sublinguale) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinalacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder nicht-wäßrigen Flüssigkeit; oder als eine ÖI-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Als weitere Wirkstoffe für die Kombinationspräparate sind geeignet:
Alle Antidiabetika, die in der Roten Liste 2001, Kapitel 12 genannt sind. Sie können mit den erfindungsgemäßen Verbindungen der Formel I insbesonders zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen.

Antidiabetika umfassen Insulin und Insulinderivate, wie z.B. Lantus^{®} oder HMR 1964, GLP-1-Derivate wie z.B. diejenigen die in WO 98/08871 von Novo Nordisk A/S offenbart wurden, sowie oral wirksame hypoglykämische Wirkstoffe.

Die oral wirksamen hypoglykämischen Wirkstoffe umfassen vorzugsweise Sulphonylfharnstoffe, Biguadine, Meglitinide, Oxadiazolidindione, Thiazolidindione, Glukosidase-Inhibitoren, Glukagon-Antagonisten, GLP-1-Agonisten, Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen wie antihyperlididämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, Gl 262570, verabreicht.

Bei einer Ausführungsform der Erfindung werden die Verbindungen der Formel I in Kombination mit PPAR alpha Agonist, wie z.B. GW 9578, GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, verabreicht.

Bei einer Ausführungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Bay 13-9952, BMS-201038, R-103757, verabreicht.

Bei einer Ausführungsform der Erfindung werden die Verbindungen der Formel I in Kombination mit Gallensäureresorptionsinhibitor , wie z.B. HMR 1453, verabreicht.

Bei einer Ausführungsform der Erfindung werden die Verbindungen der Formel I in Kombination mit einem CETP-Inhibitor, wie z.B. Bay 194789, verabreicht.

Bei einer Ausführungsform der Erfindung werden die Verbindungen der Formel I in Kombination mit einem polymeren Gallensäureadsorber, wie z.B. Cholestyramin, Colesolvam, verabreicht.

Bei einer Ausführungsform der Erfindung werden die Verbindungen der Formel I in Kombination mit einem LDL-Rezeptorinducer, wie z.B. HMR1171, HMR1586, verabreicht.

Bei einer Ausführungsform der Erfindung werden die Verbindungen der Formel I in Kombination mit einem ACAT-Inhibitor, wie z.B. Avasimibe, verabreicht.

Bei einer Ausführungsform der Erfindung werden die Verbindungen der Formel 1 in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen synthetase inhibitor, wie z.B. BMS-188494, verabreicht.

Bei einer Ausführungsform der Erfindung werden die Verbindungen der Formel I in Kombination mit einem Lipoprotein(a) antagonist, wie z.B. Cl-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.

Bei einer Ausführungsform werden die Verbindungen der Formel I in Kombination mit einem Sulphonylharnstoff, wie z.B. Tolbutamid, Glibendamid, Glipizid oder Gliclazid, verabreicht.

Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.

Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform werden die Verbindungen der Formel I in Kombination mit einem α-Glukosidase-Inhibitor, wie z.B. Miglitol oder Acarbose, verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Gliazid oder Repaglinid.

Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormon, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten verabreicht.

Bei einer Ausführungsform der Erfindung ist der weitere Wirkstoff Leptin.

Bei einer Ausführungsform ist der weitere Wirkstoff Dexamphatamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin.

Bei einer Ausführungsform ist der weitere Wirkstoff Orlistat.

Bei einer Ausführungsform ist der weitere Wirkstoff Mazindol oder Phentermin.

Bei einer Ausführungsform werden die Verbindungen der Formel I in Kombination mit Ballaststoffen, vorzugsweise unlöslichen Ballaststoffen, wie z.B. Caromax^{®} verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden. Die Kombination von Verbindungen der Formel I mit Caromax^{®} zeichnet sich neben einer Wirkverbesserung, insbesonders in der LDL-Cholesterinsenkung, gegenüber den Einzelwirkstoffen, auch durch Ihre verbesserte Verträglichkeit aus.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

Gegenstand der Erfindung sind weiterhin sowohl Stereoisomerengemische der Formel I, als auch die reinen Stereoisomere der Formel I, sowie Diastereomerengemische der Formel I als auch die reinen Diastereomere. Die Trennung der Gemische erfolgt auf chromatographischem Weg.

Bevorzugt sind racemische als auch enantiomerenreine Verbindungen der Formel I mit folgender Struktur:

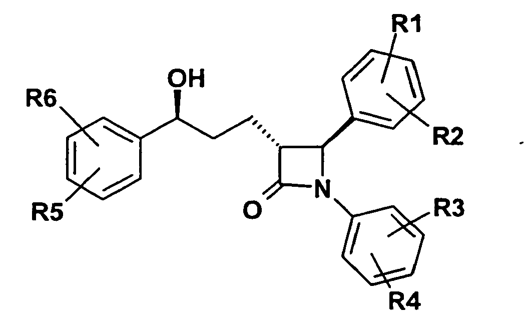

Unter Zuckerresten werden Verbindungen verstanden, die sich von Aldosen und Ketosen mit 3 bis 7 Kohlenstoffatomen ableiten, die der D- oder L-Reihe angehören können: dazu gehören auch Aminozucker, Zuckeralkohole oder Zuckersäuren. Beispielhaft seien genannt Glucose, Mannose, Fructose, Galaktose, Ribose, Erythrose, Glycerinaldehyd, Sedoheptulose, Glucosamin, Galaktosamin, Glucuronsäure, Galakturonsäure, Gluconsäure, Galaktonsäure, Mannonsäure, Glucamin, 3-Amino-1,2-propandiol, Glucarsäure und Galaktarsäure.

Mit Dizucker sind Saccharide gemeint, die aus zwei Zuckereinheiten bestehen. Di-, Tri-, oder Tetrasaccharide entstehen durch acetalartige Bindung von 2 oder mehreren Zuckern. Die Bindungen können dabei in der α- oder β-Form auftreten. Beispielhaft seien genannt Laktose, Maltose und Cellobiose.

Wenn der Zucker substituiert ist, so erfolgt die Substitution bevorzugt am Wasserstoffatom einer OH-Gruppe des Zuckers.

Für die Hydroxygruppen der Zucker kommen im wesentlichen folgende Schutzgruppen in Frage: Benzyl-, Acetyl-, Benzoyl-, Pivaloyl-, Trityl-, tert.-Butyldimethylsilyl-, Benzyliden-, Cyclohexyliden- oder lsopropylidenschutzgruppen.

Die Erfindung betrifft weiterhin ein Verfahren zur Herstellung von Diphenylazetidinonderivaten der Formel I. 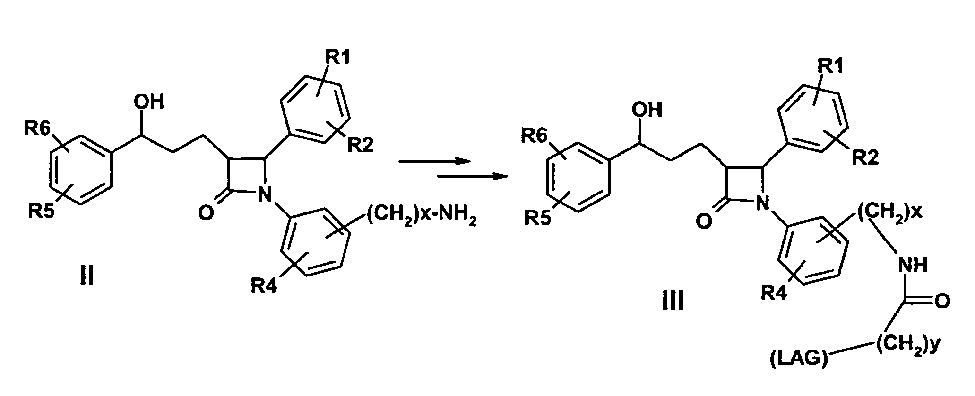 x und y können unabhängig voneinander 0 bis 10 bedeuten. Die Verknüpfung von -(CH₂)x-NH₂ in Verbindung II kann alternativ auch an einem der anderen beiden Phenylringen sein.

Das Verfahren zur Herstellung der Verbindungen der Formel I ist dadurch gekennzeichnet, daß man ein Amin der Formel II mit einem Alkylierungs- oder einem Acylierungsreagenz umsetzt, das bevorzugt in omega-Position eine weitere Funktionalität - evtl. in geschützter Form - trägt. Diese wird (nach Entschützung) zur Anknüpfung der (LAG) verwendet, beispielsweise unter Ausbildung von Ether-, Amin oder Amidbindungen.

Die nachfolgenden Beispiele dienen zur näheren Erläuterung der Erfindung, ohne dieselbe auf in den Beispielen beschriebene Produkte und Ausführungsformen einzuschränken.

### Beispiel I

### 5-(2,3,4,5,6-Pentahydroxy-hexylamino)-pentansäure-4-[3-(3-hydroxy-3-phenylpropyl)-2- (4-methoxy-phenyl)-4-oxo-azetidin-1-yl]-benzylamid (3)

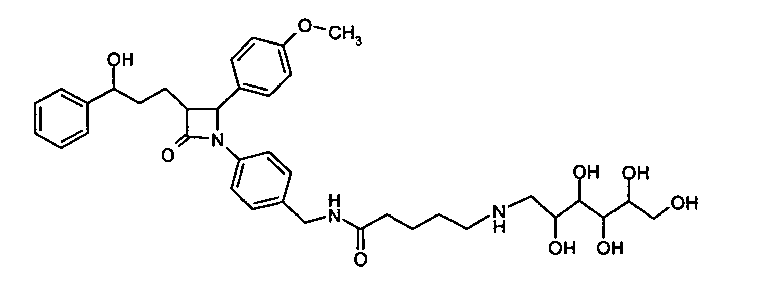

### a) 5-Bromo-pentansäure- 4-[3-(3-hydroxy-3-phenyl-propyl)-2-(4-methoxy-phenyl)-4-oxo- azetidin-1-yl]-benzylamid (2)

416 mg 1-(4-Aminomethyl-phenyl)-3-(3-hydroxy-3-phenyl-propyl)-4-(4-methoxyphenyl)-azetidin-2-on (1) werden in 10 ml trockenem Dichlormethan gelöst und mit 0.2 ml Triethylamin versetzt. Unter Eiskühlung gibt man 200 mg 5-Bromvalerylchlorid gelöst in 2ml Dichlormethan dazu und rührt 5 Stunden bei Raumtemperatur. Man versetzt mit 5 ml Wasser, säuert mit 0.5 N HCl an (pH~ 3) trennt die Phasen, wäscht die wässriger Phase mit wenig Dichlormethan, trocknet die vereinten organischen Lösungen mit Natriumsulfat und reinigt den Rückstand nach Entfernen des Lösemittels durch Säulenfiltration an Kieselgel. Man erhält 2 als Öl mit dem Molekulargewicht 579.54 (C₃₁H₃₅BrN₂O₄) MS (FAB): 581/579 (M+H⁺).

### b) 5-(2,3,4,5,6-Pentahydroxy-hexylamino)-pentansäure-4-[3-(3-hydroxy-3-phenylpropyl)-2- (4-methoxy-phenyl)-4-oxo-azetidin-1-yl]-benzylamid (3)

300 mg 2 werden in 10 ml Dimethformamid gelöst und mit 191 mg 6-Amino-hexan-1,2,3,4,5-pentanol versetzt. Man rührt bei 80° C bis die Umsetzung kontrolliert durch Dünnschichtchromatographie weitgehend beendet ist (nach etwa 2 Stunden). Danach wird das Lösemittel im Vakuum entfernt und der Rückstand über Kieselgel chromatographiert (Laufmittel: CH₂Cl₂ / Methanol / konz. Ammoniak = 30:10:2). Man erhält 3 mit dem Molekulargewicht 679.82 (C₃₇H₄₉N₃O₉); MS (FAB): 680 (M+H⁺).

### Beispiel II

### 2,3,4,5,6-Pentahydroxy-hexansäure 4-{1-(4-fluor-phenyl)-3-[3-(4-fluor-phenyl)-3-hydroxy-propyl]-4-oxo-azetidin-2-yl}-benzylamid (4)

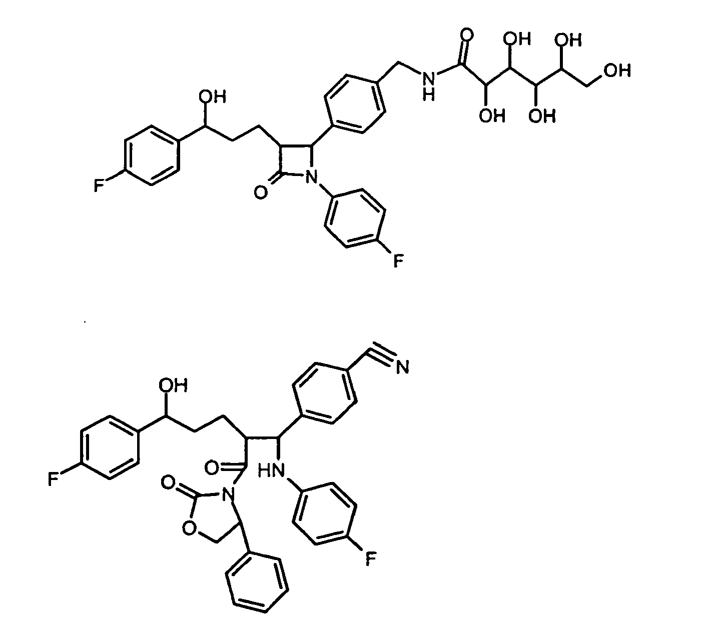

### a) 4-[5-(4-Fluor-phenyl)-1-(4-fluor-phenylamino)-5-hydroxy-2-(2-oxo-4-phenyloxazolidin-3- carbonyl)-pentyl]-benzonitril (5)

2.5 g 3-[5-(4-Fluor-phenyl)-5-hydroxy-pentanoyl]-4-phenyl-oxazolidin-2-on werden in 30 ml Dichlormethan unter Argon gelöst, dazu gibt man 3.9 g 4-[(4-Fluor-phenylimino)-methyl]-benzonitril und kühlt auf -10°C.Zu dieser Mischung gibt man 6.4 ml Diisopropylethylamin und innerhalb von 30 min 4.05 ml Trimethylsilylchlorid, so dass die Temperatur -5°C nicht übersteigt. Bei dieser Temp. wird 1 Std. nachgerührt und dann auf -25°C gekühlt. Dann werden 0.8 ml Titantetrachlorid langsam zugegeben. Die dunkle Mischung wird über Nacht bei - 25 bis -30°C gerührt danach mit 35 ml 7proz. Weinsäurelösung zersetzt und 1 Std. bei Raumtemp. nachgerührt. Anschließend gibt man 15 ml einer 20proz. Natriumhydrogencarbonatlösung dazu und rührt erneut 1 Std. Nach Phasentrennung wird die org. Phase mit 30 ml Waser gewaschen, über Magnesiumsulfat getrocknet und auf ca. 10 ml eingeengt. Nach Zugabe von 2 ml Bistrimethylsilylacetamid erwärmt man 30 min. zum Rückfluss und engt danach i.Vak. ein. Der Rückstand wir d mit Ethylacetat/Heptan zur Kristallisation gebracht. Man saugt ab und trocknet i.Vak. Man erhält 5 mit dem Molekulargewicht 653.81 (C₃₇H₃₇F₂N₃O₄Si); MS (ESI+): 654.3 (M+H⁺), 582.2 (M+H⁺-Si(CH₃)₃).

### b) {1-(4-Fluor-phenyl)-3-[3-(4-fluor-phenyl)-3-hydroxy-propyl]-4-oxo-azetidin-2-yl}-benzonitril (6)

2 g 5 werden in 20 ml Methyl-tert.-butyl-ether gelöst und mit 100 mg Tetrabutylammoniumfluorid-Trihydrat und 1.3 ml Bistrimethylsilylacetamid ca. 1 h auf 40°C erwärmt. Man verfolgt die Reaktion im Dünnschichtchromatogramm. Nach beendeter Umsetzung setz man zunächst 0.2 ml Eisessig zu , rührt 30 min und engt ein. Der Rückstandwird mit 20ml einer Mischung von Isopropanol /2N Schwefelsäure = 10:1 versetzt und 1 Std. gerührt. Nach Zugabe einer Spatelspitze festem Natriumhydrogencarbonat engt man erneut i. Vak. ein, nimmt mit Ethylacetat auf, wäscht die org. Phase mit Wasser, trocknet und reinigt nach Entfernen des Lösemittels den Rückstand durch Säulenchromatographie (SiO₂, CH₂Cl₂/Methanol = 100: 1). Man erhält 6 mit dem Molekulargewicht 418.45 (C₂₅H₂₀F₂N₂O₂); MS (DCI+): 419 (M+H⁺).

### c) 4-(4-Aminomethyl-phenyl)-1-(4-fluor-phenyl)-3-[3-(4-fluor-phenyl)-3-hydroxypropyl]- azetidin-2-on (7)

200 mg 6 werden in 20 ml Ethanol gelöst und mit 0.5 ml konz. Ammoniak über Raney-Nickel 30 Std bei 75 bar Wasserstoff und 25°C hydriert. Man saugt vom Katalysator ab, engt i. Vak. ein und reinigt den Rückstand durch Säulenfiltration (SiO₂, CH₂Cl₂/Methanol/. NH₃ conc = 100:10:1). Man erhält 7 mit dem Molekulargewicht 422.5 (C₂₅H₂₂F₂N₂O₂); MS (DCI+): 423 (M+H⁺), 405 (M+H⁺- H₂O).

### d) 2,3,4,5,6-Pentahydroxy-hexansäure 4-{1-(4-fluor-phenyl)-3-[3-(4-fluor-phenyl)-3-hydroxy-propyl]-4-oxo-azetidin-2-yl}-benzylamid (4)

50 mg 7 und 25 mg 3,4,5-Trihydroxy-6-hydroxymethyl-tetrahydro-pyran-2-on werden in 5 ml Methanol gelöst und mit 10 mg Na₂CO₃ über Nacht gerührt. Man saugt ab, engt i. Vak. ein und reinigt den Rückstand durch Säulenfiltration (SiO₂, CH₂Cl₂/Methanol= 10: 1). Man erhält 4 mit einem Schmelzpunkt über 180°C und dem Molekulargewicht 600.6 (C₃₁H₃₄F₂N₂O₈); MS (ESI+): 601 (M+H⁺), 583 (M+H⁺- H₂O).

### Beispiel III

### 12-(2,3,4,5,6-Pentahydroxy-hexanoylamino)-dodecansäure-4-[3-[3-(4-fluor-phenyl)-3- hydroxy-propyl]-2-(4-methoxy-phenyl)-4-oxo-azetidin-1-yl]-benzylamid (8)

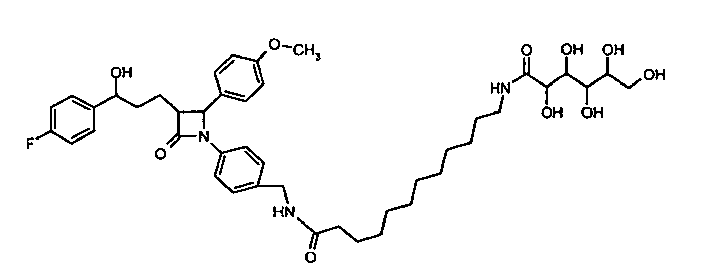

### a) 12-(2,3,4,5,6-Pentahydroxy-hexanoylamino)-dodecansäure (9)

3.5 g 12-Aminododecansäure werden in 500 ml Methanol gelöst und mit 2.7 g fein gepulvertem Natriumcarbonat und 4.8 g 3,4,5-Trihydroxy-6-hydroxymethyltetrahydro-pyran-2-on 30 Std. bei Raumtemp. gerührt. Man filtriert ab, engt ein und löst den Rückstand in 70 ml Wasser. Unter Eiskühlung gibt man schrittweise 1 N Salzsäure hinzu, bis der pH-Wert bei 1-2 liegt (ca. 50 - 55 ml). Die freie Säure fällt aus, man saugt ab und wäscht mit wenig kaltem Wasser nach und trocknet im Feinvakuum bei 35°C. Man erhält 9 mit dem Molekulargewicht 393.48 (C₁₈H₃₅NO₈); MS (ESI+): 394 (M+H⁺); (ESI-):392 (M-H)⁻.

### b) 12-(2,3,4,5,6-Pentahydroxy-hexanoylamino)-dodecansäure-4-[3-[3-(4-fluorphenyl)-3- hydroxy-propyl]-2-(4-methoxy-phenyl)-4-oxo-azetidin-1-yl]-benzylamid (8)

Wird analog zu Beispiel II hergestellt, ausgehend von 1-(4-Aminomethyl-phenyl)-3-(3-hydroxy-3-phenyl-propyl)-4-(4-methoxy-phenyl)-azetidin-2-on. Man erhält 12-(2,3,4,5,6-Pentahydroxy-hexanoylamino)-dodecansäure 4-[3-(3-hydroxy-3-phenyl- propyl)-2-(4-methoxy-phenyl)-4-oxo-azetidin-1-yl]-benzylamid mit dem Schmelzpunkt 100°C und dem Molekulargewicht 792 (C₄₄H₆₁N₃O₁₀); MS(ESI+): 792 (M+H⁺).

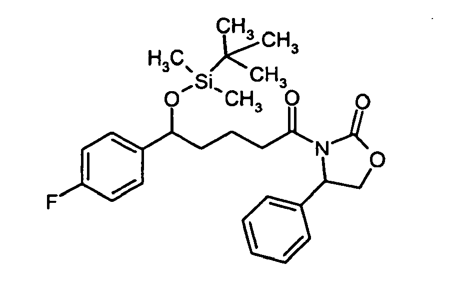

### 3-[5-(tert-Butyl-dimethyl-silanyloxy)-5-(4-fluor-phenyl)-pentanoyl]-4-phenyl-oxazolidin-2-on (10)

30 g 3-[5-(4-Fluor-phenyl)-5-hydroxy-pentanoyl]-4-phenyl-oxazolidin-2-on werden in 50 ml DMF gelöst. Nach Zugabe von 14.3 g Imidazol und 19 g tert.-Butyl-dimethylsilylchlorid in 25 ml DMF wird bis zur vollständigen Umsetzung bei Raumtemperatur gerührt (2-4 h). Die Reaktionslösung wird eingeengt, mit Wasser versetzt und mit Essigsäureethylester extrahiert. Nach Trocknen der organischen Phase über Magnesiumsulfat und Einengung erhält man 10: C₂₆H₃₄FNO₄Si (471.65) MS (ESI) 494 (M + Na)

### 3-[(4-Fluor-phenylimino)-methyl]-benzonitril (11)

Zu 12 g meta-Cyano-benzaldehyd in 60 ml Isopropanol werden 88 ml para-Fluoranilin zugetropft. Nach 1 h bei 60°C fällt das Produkt aus. Man läßt auf Raumtemperatur kommen filtriert ab und wäscht den Rückstand mit Isopropanol. Nach Trocknung erhält man 11 mit dem Schmp. 101°C. C₁₄H₉FN₂ (224.24).

### 3-[5-(tert-Butyl-dimethyl-silanyloxy)-5-(4-fluor-phenyl)-1-(4-fluor-phenylamino)-2-(2-oxo-4-phenyl-oxazolidin-3-carbonyl)-pentyl]-benzonitril (12)

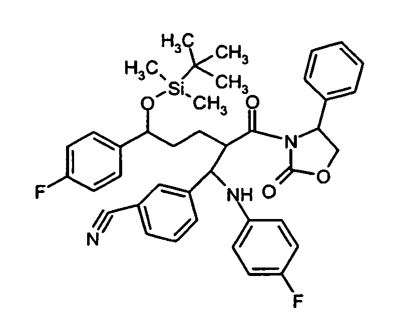

Zu 14 g 3-[5-(tert-Butyl-dimethyl-silanyloxy)-5-(4-fluor-phenyl)-pentanoyl]-4-phenyl-oxazolidin-2-on (10) und 12.5 g 3-[(4-Fluor-phenylimino)-methyl]-benzonitril 11 in 200 ml Methylenchlorid werden bei 10°C 24 ml Diisopropylethylamin zugegeben und 7.1 ml Trimethylsilylchlorid zugetropft. Nach 1 h werden bei -10°C 3.4 ml Titantetrachlorid zugetropft. Es wird 3 h bei -10°C gerührt und weitere 12 h bei-30°C stehen gelassen. Anschließend wird mit 8 ml Essigsäure und 140 ml einer 7% wässrigen Weinsäurelösung versetzt und weitere 2 h bei Raumtemperatur gerührt. Nach Zugabe von 50 ml 20% wässriger Natriumhydrogensulfitlösung wird noch mal 1 h gerührt und mit Methylenchlorid extrahiert. Die organische Phase wird über Magnesiumsulfat getrocknet, eingeengt und durch Chromatographie an Kieselgel /Ethylacetat/Heptan = 1/3 -> 1/1 gereinigt). Man erhält 12
C₄₀H₄₃F₂N₃O₄Si (695.89) MS (ESI) 696 (M + H)

### 3-[3-[3-(tert-Butyl-dimethyl-silanyloxy)-3-(4-fluor-phenyl)-propyl]-1-(4-fluor- phenyl)-4-oxo-azetidin-2-yl]-benzonitril (13)

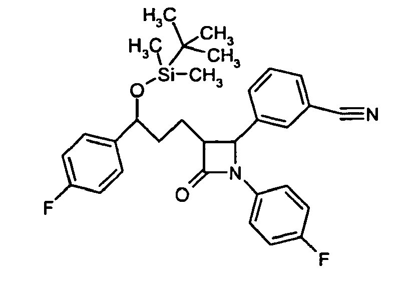

Eine Mischung aus 13 g 3-[5-(tert-Butyl-dimethyl-silanyloxy)-5-(4-fluor-phenyl)-1-(4-fluor-phenylamino)-2-(2- oxo-4-phenyl-oxazolidin-3-carbonyl)-pentyl]-benzonitril 12, 50 ml Bistrimethylsilylacetamid, 0.5 g Tetrabutylammoniumfluorid und 100 ml tert-Butylmethylether wird unter Argon 10 h bei Raumtemperatur gerührt. Nach beendeter Reaktion werden langsam unter Eiskühlung 5 ml Essigsäure zugegeben und eingeengt. Der Rückstand wird durch Chromatographie an Kieselgel (Ethylacetat/Heptan = 1/8) getrennt. Man erhält 13:
C₃₁H₃₄F₂N₂O₂Si (532.71) MS (ESI) 555 (M + Na)

### 3-{1-(4-Fluor-phenyl)-3-[3-(4-fluor-phenyl)-3-hydroxy-propyl]-4-oxo-azetidin-2-yl}-benzonitril (14)

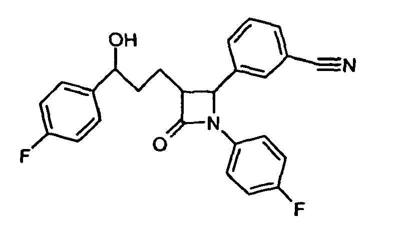

Zu 7.8 g 3-[3-[3-(tert-Butyl-dimethyl-silanyloxy)-3-(4-fluor-phenyl)-propyl]-1-(4-fluorphenyl)-4-oxo-azetidin-2-yl]-benzonitril (13) in 200 ml Methanol werden 10 ml 1N Salzsäure gegeben und 12 h gerührt. Die Reaktion wird mit wässriger Natriumhydrogencarbonatlösung versetzt und mit Methylenchlorid extrahiert. Die organische Phase wird über Magnesiumsulfat getrocknet, eingeengt und über eine Chromatographie an Kieselgel (Ethylacetat/Heptan = 1/3 -> 1/1)gereinigt. Man erhält 14 : C₂₅H₂₀F₂N₂O₂ (418.45) MS (ESI) 401 (M + H - H₂O)

### Beispiel V

### 2,3,4,5,6-Pentahydroxy-hexansäure-3-{2-(4-fluor-phenyl)-4-[3-(4-fluor-phenyl)-3-hydroxy-propyl]- 4-oxo-azetidin-2-yl }-benzylamid (16)

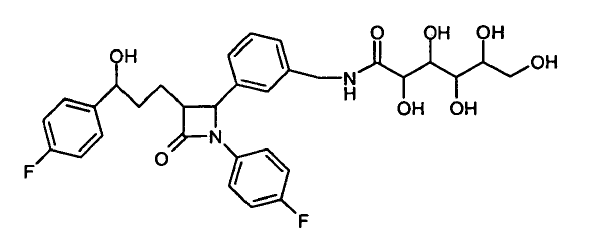

Zu einer Lösung von 100 mg 4-(3-Aminomethyl-phenyl)-1-(4-fluor-phenyl)-3-[3-(4-fluor-phenyl)-3-hydroxy-propyl]- azetidin-2-on 6 und 46 mg 3,4,5-Trihydroxy-6-hydroxymethyl-tetrahydro-pyran-2-on in 5 ml Methanol werden 25 mg Natriumcarbonat gegeben und bis zur vollständigen Umsetzung bei Raumtemperatur gerührt. Die Reaktionslösung wird filtriert und eingeengt. Der Rückstand wird über HPLC (Knauer Eurospher-100-10-C18, Wasser (0.1 % Trifluoressigsäure)/Acetonitril (0.1% Trifluoressigsäure) = 80/20 -> 10/90) gereinigt. Man erhält 16 :
C₃₁H₃₄F₂N₂O₈ (600.62) MS (ESI) 601 (M + H)

### Beispiel VIII

### 2,3,4,5,6-Pentahydroxy-hexansäure [5-(3-{1-(4-fluoro-phenyl)-3-[3-(4-fluor-phenyl)-3-hydroxy-propyl]-4-oxo-azetidin-2-yl}-phenylcarbamoyl)-pentyl]-amid (19)

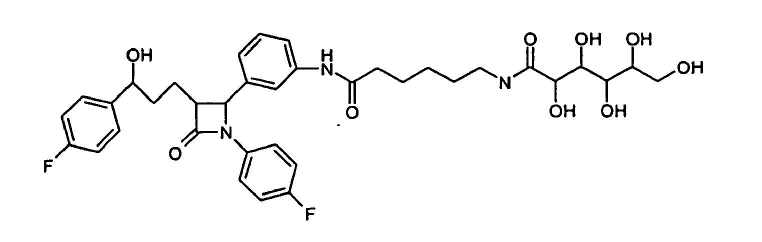

19 wird analog zu 18 ausgehend von 100 mg 4-(3-Aminomethyl-phenyl)-1-(4-fluorphenyl)-3-[3-(4-fluor-phenyl)-3-hydroxy-propyl]- azetidin-2-on 15 , 108 mg 6-(2,3,4,5,6-Pentahydroxy-hexanoylamino)-hexansäure, 93 µl Diisopropylcarbodiimid, 65 mg Hydroxybenzotriazol in 2 ml Methylenchlorid hergestellt. Man erhält 10:
C₃₇H₄₅F₂N₃O₉ (713.78) MS (ESI) 714 (M + H)

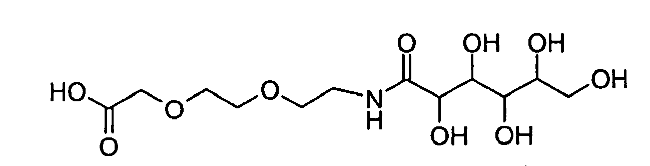

### {2-[2-(2,3,4,5,6-Pentahydroxy-hexanoy)amino)-ethoxy]-ethoxy}-essigsäure (20)

Zu einer Lösung von 450 mg [2-(2-Amino-ethoxy)-ethoxy]-essigsäure und 318 mg 3,4,5-Trihydroxy-6-hydroxymethyl-tetrahydro-pyran-2-on in 10 ml Methanol werden 172 mg Natriumcarbonat gegeben und bis zur vollständigen Umsetzung bei Raumtemperatur gerührt. Die Reaktionslösung wird filtriert und eingeengt. Der Rückstand wird in Wasser und Acetonitril (1/1) aufgenommen, wobei sich 2 Phasen bilden. Die wäßrige Phase wird eingeengt und enthält 20 :
C₁₂H₂₃NO₁₀ (341.32) MS (ESI) 342 (M + H)

### Beispiel IX

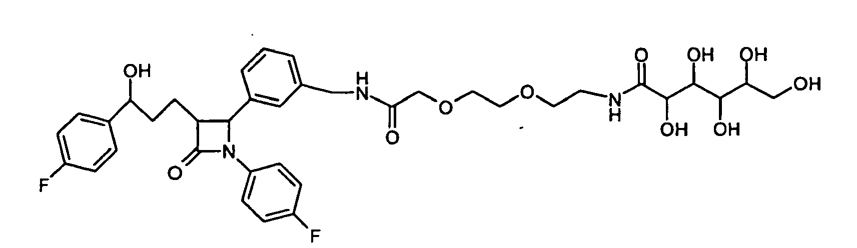

### 2,3,4,5,6-Pentahydroxy-hexansäure (2-{2-[(3-{1-(4-fluor-phenyl)-3-[3-(4-fluorphenyl)-3-hydroxy-propyl]-4-oxo-azetidin-2-yl}-benzylcarbamoyl)-methoxy]-ethoxy}-ethyl)- amid (21)

21 wird analog zu 18 ausgehend von 100 mg 4-(3-Aminomethyl-phenyl)-1-(4-fluorphenyl)-3-[3-(4-fluor-phenyl)-3-hydroxy-propyl]- azetidin-2-on (15), 122 mg {2-[2-(2,3,4,5,6-Pentahydroxy-hexanoylamino)-ethoxy]-ethoxy}-essigsäure (20), 93 µl Diisopropytcarbodiimid, 65 mg Hydroxybenzotriazol in 2 ml Dimethylformamid hergestellt. Man erhält 21 : C₃₇H₄₅F₂N₃O₁₁ (745.78) MS (ESI) 746 (M + H)

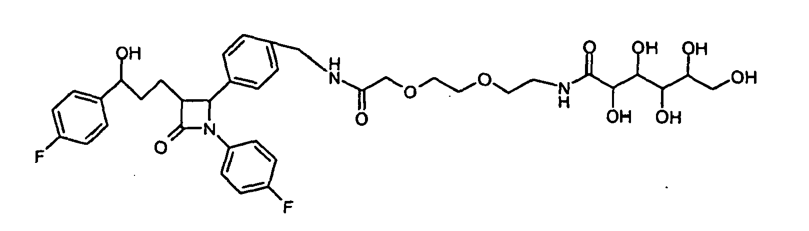

### Beispiel X

### 2,3,4,5,6-Pentahydroxy-hexansäure (2-{2-[(4-{1-(4-fluor-phenyl)-3-[3-(4-fluorphenyl)-3-hydroxy-propyl]-4-oxo-azetidin-2-yl}-benzylcarbamoyl)-methoxy]-ethoxy}-ethyl)- amid (22)

22 wird analog zu 18 ausgehend von 100 mg 4-(4-Aminomethyl-phenyl)-1-(4-fluorphenyl)-3-[3-(4-fluorophenyl)-3-hydroxy-propyl]- azetidin-2-on, 122 mg {2-[2-(2,3,4,5,6-Pentahydroxy-hexanoylamino)-ethoxy]-ethoxy}-essigsäure 20, 93 µl Düsopropylcarbodiimid, 65 mg Hydroxybenzotriazol in 2 ml Dimethylformamid und 1 ml Acetonitril hergestellt. Man erhält 22 :
C₃₇H₄₅F₂N₃O₁₁ (745.78) MS (ESI) 746 (M +H)

### Essigsäure-2,3,4-triacetoxy-1-{2-[2-(2-amino-ethoxy)-ethoxy]-acetyl}-5-hydroxy-pentyl ester (23)

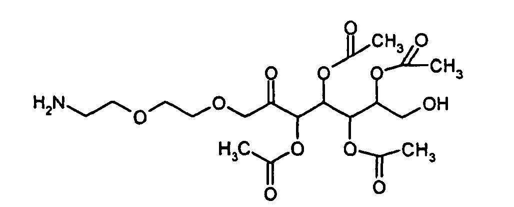

Eine Suspension aus 1.12 g Essigsäure-2,3,4-triacetoxy-1-{2-[2-(2-azido-ethoxy)-ethoxy]-acetyl}-5-hydroxy-pentylester und 1.0 g Raney-Nickel in 100 ml Ethanol werden in einer Hydrierapparatur unter Wasserstoffatmosphäre 4 h geschüttelt. Die Reaktionslösung wird filtriert und eingeengt. Der Rückstand enthält 23 :
C₂₀H₃₃NO₁₂ (479.49) MS (ESI) 480 (M + H)

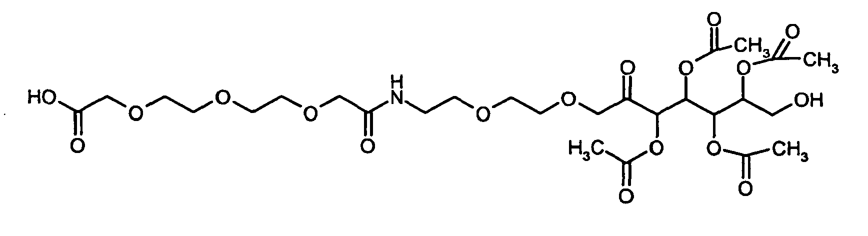

### {2-[2-({2-[2-(3,4,5,6-Tetraacetoxy-7-hydroxy-2-oxo-heptyloxy)-ethoxy]-ethylcarbamoyl}-methoxy)-ethoxy]-ethoxy}-essigsäure (24)

Eine Lösung aus 500 mg Essigsäure-2,3,4-triacetoxy-1-{2-[2-(2-amino-ethoxy)-ethoxy]-acetyl}-5-hydroxy-pentylester 23 , 1.15 g [2-(2-Carboxymethoxy-ethoxy)-ethoxy]-essigsäure, 400 µl Diisopropylcarbodiimid, 288 mg Hydroxybenzotriazol in 20 ml Methylenchlorid wird 12 h bei Raumtemperatur gerührt. Die Reaktionslösung wird eingeengt und über HPLC (Knauer Eurospher-100-10-C18, Wasser (0.1 % Trifluoressigsäure)/Acetonitril (0.1 % Trifluoressigsäure) = 80/20 -> 10/90) getrennt. Man erhält 24 : C₂₈H₄₅NO₁₈ (683.67) MS (ESI) 684 (M + H)

### [2-({2-[2-(3,4,5,6-Tetraacetoxy-7-hydroxy-2-oxo-heptytoxy)-ethoxy]-ethylcarbamoyl}-methoxy)-ethoxy]-essigsäure (25)

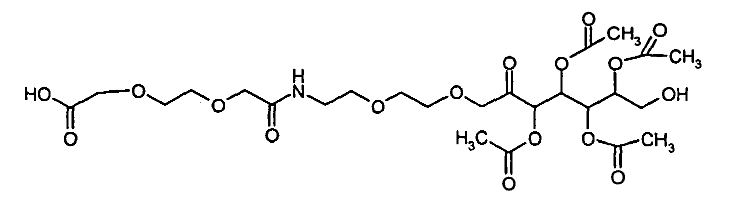

25 wird analog zu 24 ausgehend von 500 mg Essigsäure-2,3,4-triacetoxy-1-{2-[2-(2-amino-ethoxy)-ethoxy]-acetyl}-5-hydroxy-pentylester 23, 927 mg (2-Carboxymethoxy-ethoxy)-essigsäure, 400 µl Düsopropylcarbodiimid, 288 mg Hydroxybenzotriazol in 20 ml Methylenchlorid hergestellt. Man erhält 25 :
C₂₆H₄₁NO₁₇ (639.61) MS (ESI) 640 (M + H)

### {2-[2-({2-[2-(3,4,5,6,7-Pentahydroxy-2-oxo-heptyloxy)-ethoxy]-ethylcarbamoyl}-methoxy)-ethoxy]-ethoxy}-essigsäure (26)

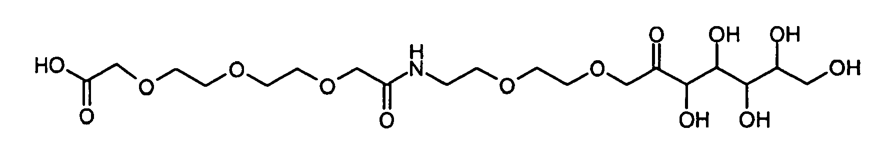

Eine Lösung von 200 mg {2-[2-({2-[2-(3,4,5,6-Tetraacetoxy-7-hydroxy-2-oxo-heptyloxy)-ethoxy]-ethylcarbamoyl}- methoxy)-ethoxy]-ethoxy}-essigsäure (24) in 5 ml Methanol wird bei Raumtemperatur mit 100 µL einer 5.4 M Natriummethanolatlösung in Methanol versetzt und 2 h gerührt. Die Reaktionslösung wird mit 1 g Amberlite IR 120 versetzt, 10 min gerührt, filtriert, eingeengt und man erhält 26 :
C₂₀H₃₇NO₁₄ (515.52) MS (ESI) 516 (M + H)

### [2-({2-[2-(3,4,5,6,7-Pentahydroxy-2-oxo-heptyloxy)-ethoxy]-ethylcarbamoyl}-methoxy)- ethoxy]-acetic acid (27)

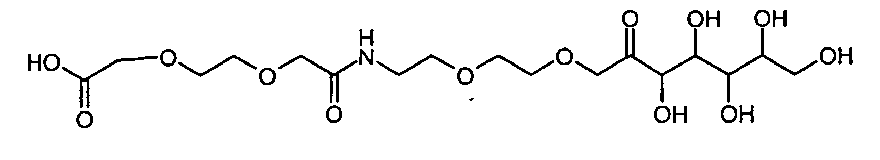

27 wird analog 26 ausgehend von 200 mg 25 hergestellt. Man erhält 27 : C₂₆H₄₁N1O₁₇ (471.46) MS (ESI) 472 (M + H)

### Beispiel XI

### N-(4-{1-(4-Fluor-phenyl)-3-[3-(4-fluor-phenyl)-3-hydroxy-propyl]-4-oxo-azetidin-2-yl)-benzyl)-2-{2-[2-({2-[2-(3,4,5,6,7-pentahydroxy-2-oxo-heptyloxy)-ethoxy]-ethylcarbamoyl}- methoxy)-ethoxy]-ethoxy}-acetamid (28)

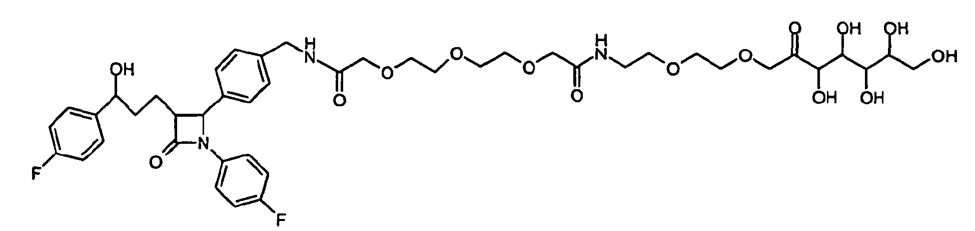

28 wird analog zu 18 ausgehend von 62 mg 4-(4-Aminomethyl-phenyl)-1-(4-fluorphenyl)-3-[3-(4-fluor-phenyl)-3-hydroxy-propyl]- azetidin-2-on, 76 mg {2-[2-({2-[2-(3,4,5,6,7-Pentahydroxy-2-oxo-heptyloxy)-ethoxy]-ethylcarbamoyl}- methoxy)-ethoxy]-ethoxy}-essigsäure 17, 57 µl Diisopropylcarbodiimid, 40 mg Hydroxybenzotriazol in 2 ml Dimethylformamid hergestellt. Man erhält 19: C₄₅H₅₉F₂N₃O₁₄ (919.98) MS (ESI) 920 (M + H)

### Beispiel XII

### N-(3-{1-(4-Fluor-phenyl)-3-[3-(4-fluor-phenyl)-3-hydroxy-propyl]-4-oxo-azetidin-2-yl}-benzyl)-2-{2-[2-({2-[2-(3,4,5,6,7-pentahydroxy-2-oxo-heptyloxy)-ethoxy]-ethylcarbamoyl}- methoxy)-ethoxy]-ethoxy}-acetamid (29)

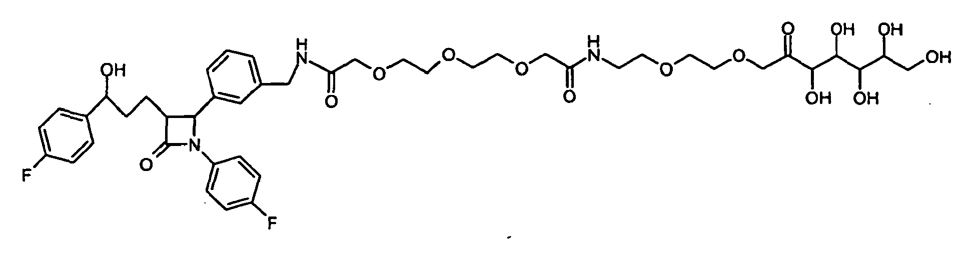

29 wird analog zu 18 ausgehend von 62 mg 4-(3-Aminomethyl-phenyl)-1-(4-fluorphenyl)-3-[3-(4-fluor-phenyl)-3-hydroxy-propyl]- azetidin-2-on 15 , 76 mg {2-[2-({2-[2-(3,4,5,6,7-Pentahydroxy-2-oxo-heptyloxy)-ethoxy]-ethylcarbamoyl}-methoxy)-ethoxy]-ethoxy}-essigsäure 26 , 57 µl Diisopropylcarbodiimid, 40 mg Hydroxybenzotriazol in 2 ml Dimethylformamid hergestellt. Man erhält 29 :
C₄₅H₅₉F₂N₃O₁₄ (919.98) MS (ESI) 920 (M + H)

### Beispiel XIII

### N-(4-{1-(4-Fluor-phenyl)-3-[3-(4-fluor-phenyl)-3-hydroxy-propyl]-4-oxo-azetidin-2-yl}-benzyl)-2-[2-({2-[2-(3,4,5,6,7-pentahydroxy-2-oxo-heptyloxy)-ethoxy]-ethylcarbamoyl}- methoxy)-ethoxy]-acetamid (30)

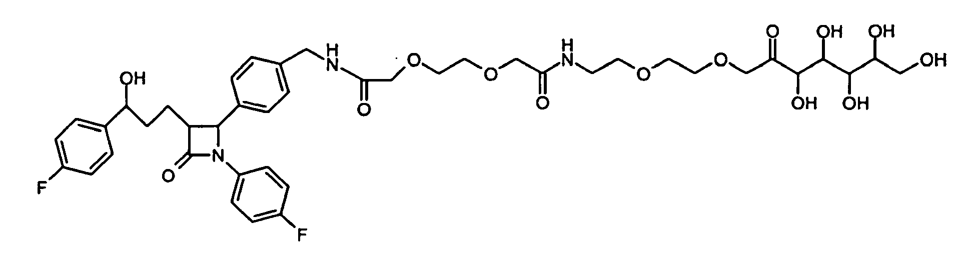

30 wird analog zu 18 ausgehend von 68 mg 4-(4-Aminomethyl-phenyl)-1-(4-fluorphenyl)-3-[3-(4-fluor-phenyl)-3-hydroxy-propyl]- azetidin-2-on, 76 mg [2-({2-[2-(3,4,5,6,7-Pentahydroxy-2-oxo-hepty)oxy)-ethoxy]-ethylcarbamoyl}-methoxy)-ethoxy]-essigsäure (27), 62 µl Diisopropylcarbodiimid, 44 mg Hydroxybenzotriazol in 2 ml Dimethylformamid hergestellt. Man erhält 30: C₄₃H₅₅F₂N₃O₁₄ (875.93) MS (ESI) 876 (M + H)

### Beispiel XIV

### N-(3-{1-(4-Fluor-phenyl)-3-[3-(4-fluor-phenyl)-3-hydroxy-propyl]-4-oxo-azetidin-2-yl}-benzyl)-2-[2-({2-[2-(3,4,5,6,7-pentahydroxy-2-oxo-heptyloxy)-ethoxy]-ethylcarbamoyl}- methoxy)-ethoxy]-acetamid (31)

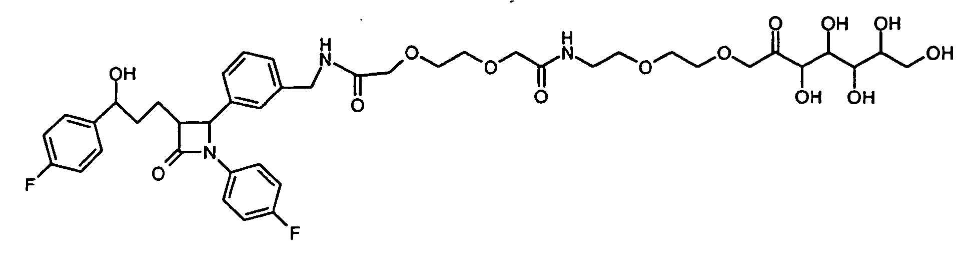

31 wird analog zu 18 ausgehend von 68 mg 4-(3-Aminomethyl-phenyl)-1-(4-fluorphenyl)-3-[3-(4-fluor-phenyl)-3-hydroxy-propyl]- azetidin-2-on (15), 76 mg [2-({2-[2-(3,4,5,6,7-Pentahydroxy-2-oxo-heptyloxy)-ethoxy]-ethylcarbamoyl}-methoxy)-ethoxy]-essigsäure (27), 62 µl Diisopropylcarbodiimid, 44 mg Hydroxybenzotriazol in 2 ml Dimethylformamid hergestellt. Man erhält 31 : C₄₃H₅₅F₂N₃O₁₄ (875.93) MS (ESI) 876 (M + H)

### Beispiel XVII

### Dodecyl-[10-(4-{1-(4-fluor-phenyl)-3-[3-(4-fluor-phenyl)-3-hydroxy-propyl]-4-oxo-azetidin-2-yl}-benzylcarbamoyl)-decyl]-dimethyl-ammonium trifluoracetat (34)

Die Verbindung des Beispiels XVII wird wie die des Beispiels XV gewonnen mit dem Unterschied, dass statt (3-Carboxy-propyl)-trimethyl-ammonium chlorid das (10-Carboxy-decyl)-dodecyl-dimethyl-ammonium chlorid eingesetzt wird. Man erhält Dodecyl-[10-(4-{1-(4-fluor-phenyl)-3-[3-(4-fluor-phenyl)-3-hydroxy-propyl]-4-oxo-azetidin-2-yl}-benzylcarbamoyl)-decyl]-dimethyl-ammonium trifluoracetat mit dem Molekulargewicht von 803,16 (C₅₀H₇₄F₂N₃O₃; Kation); MS (ESI): 803.77 (M⁺).

### Beispiel XVIII

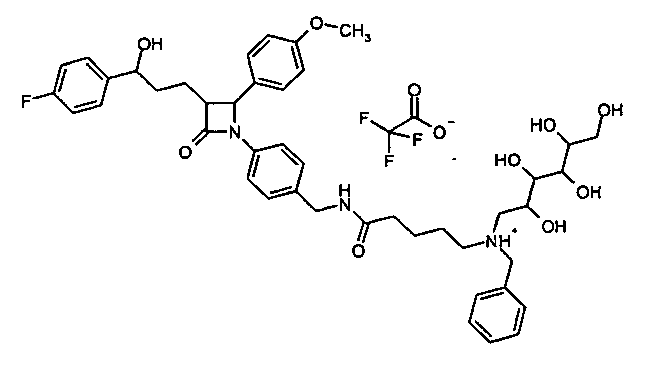

### Benzyl-(4-{4-[3-[3-(4-fluor-phenyl)-3-hydroxy-propyl]-2-(4-methoxy-phenyl)-4-oxo-azetidin-1-yl]-benzylcarbamoyl}-butyl)-(2,3,4,5,6-pentahydroxy-hexyl)-ammonium; trifluoracetat (35)

### a) 5-[Benzyl-(2,3,4,5,6-pentahydroxy-hexyl)-amino]-pentansäuremethylester (36)

1,37 g 6-Benzylamino-hexan-1,2,3,4,5-pentanol werden bei Raumtemperatur in 30 ml trockenem Dimethylformamid suspendiert, mit 1,45 g Kaliumcarbonat, 0,83 g Kaliumjodid und 0,86 ml 5-Bromvaleriansäuremethylester versetzt und über Nacht bei Raumtemperatur gerührt. Am folgenden Tag wird die Reaktionsmischung filtriert und das Filtrat wird im Vakuum eingeengt und zur Reinigung einer Chromatographie unterworfen (Kieselgel; Essigsäureethylester/Methanol/Wasser 5/1/0,1). Man erhält 5-[Benzyl-(2,3,4,5,6-pentahydroxy-hexyl)-amino]-pentansäuremethylester mit dem Molekulargewicht 385,46 (C₁₉H₃₁NO₇); MS (ESI): 386.33 (M+H⁺).

### b) 5-[Benzyl-(2,3,4,5,6-pentahydroxy-hexyl)-amino]-pentansäure (37)

0,46 g 5-[Benzyl-(2,3,4,5,6-pentahydroxy-hexyl)-amino)-pentansäuremethylester werden bei Raumtemperatur in einer Mischung aus 5 ml Ethanol und 5 ml Wasser gelöst, mit 0,4 g Kaliumhydroxid versetzt und 2 h bei 80°C gerührt. Danach wird die abgekühlte Reaktionsmischung im Vakuum eingeengt, der Rückstand in Wasser aufgenommen, mit Salzsäure neutralisiert und erneut eingeengt. Das Rohprodukt wird in Ethanol suspendiert; die Suspension filtriert und das Filtrat im Vakuum eingeengt. Man erhält 5-[Benzyl-(2,3,4,5,6-pentahydroxy-hexyl)-amino]-pentansäure mit dem Molekulargewicht 371,43 (C₁₈H₂₉NO₇); MS (ESI): 372.2 (M+H⁺).

### c) 3-[5-(tert-Butyl-dimethyl-silanyloxy)-5-(4-fluor-phenyl)-pentanoyl]-4-phenyl-oxazolidin-2-on (38)

27 g 3-[5-(4-Fluor-phenyl)-5-hydroxy-pentanoyl]-4-phenyl-oxazolidin-2-on werden mit 13,6 g tert-Butyl-Dimethylsilylchlorid und 10,2 g Imidazol in 36 ml Dimethylformamid gelöst und 90 min. bei 60°C gerührt. Nach Beendigung der Reaktion wird das Gemisch in Essigsäureethylester gelöst und zweimal mit Wasser ausgeschüttelt. Die organische Phase wird über Magnesiumsulfat getrocknet, filtriert und im Vakuum eingeengt. Man erhält 3-[5-(tert-Butyl-dimethyl-silanyloxy)-5-(4-fluor-phenyl)-pentanoyl]-4-phenyl- oxazolidin-2-on mit dem Molekulargewicht 471,65 (C₂₆H₃₄FNO₄Si); MS (ESI): 340.28 (MH⁺- HOSi(CH₃)₂C(CH₃)₃).

### d) 4-[5-(tert-Butyl-dimethyl-silanyloxy)-5-(4-fluor-phenyl)-1-(4-methoxy-phenyl)-2-(2-oxo- 4-phenyl-oxazolidin-3-carbonyl)-pentylamino]-benzonitril (39)

16,2 g 3-[5-(tert-Butyl-dimethyl-silanyloxy)-5-(4-fluor-phenyl)-pentanoyl]-4-phenyl-oxazolidin-2-on werden in 350 ml Dichlormethan gelöst. Die Lösung wird mit 19,8 ml Hünig Base und mit 10,14 g 4-[(4-Methoxy-phenylimino)-methyl]-benzonitril versetzt und auf -10°C gekühlt. Zur gekühlten Lösung fügt man 8,52 ml Trimethylsilyltriflat hinzu und rührt 30 min. bei -10°C. Die Lösung wird nun auf -30°C abgekühlt, und es werden 44 ml Titantetrachloridlösung zugegeben. Die Reaktionsmischung wird 2 h bei -30 bis-40°C gerührt. Danach lässt man die Lösung sich auf Raumtemperatur erwärmen, wäscht die Reaktionslösung nacheinander mit 200 ml 2N Schwefelsäure, 300 ml 20%iger Natriumhydrogensulfitlösung und ges. Kochsalzlösung. Die organische Phase wird über Magnesiumsulfat getrocknet, im Vakuum eingeengt und der Rückstand wird über Kieselgel mit n-Heptan/Essigsäureethylester 3/1 gereinigt. Man erhält 4-[5-(tert-Butyl-dimethyl-silanyloxy)-5-(4-fluor-phenyl)-1-(4-methoxyphenyl)-2-(2-oxo- 4-phenyl-oxazolidin-3-carbonyl)-pentylamino]-benzonitril mit dem Molekulargewicht 707,93 (C₄₁H₄₆FN₃O₅Si); MS (ESI): 590.51 (MH⁺- C₇H₅N₂).

### e) 4-[3-[3-(tert-Butyl-dimethyl-silanyloxy)-3-(4-fluor-phenyl)-propyl]-2-(4-methoxyphenyl)-4-oxo-azetidin-1-yl]-benzonitril (40)

13,2 g 4-[5-(tert-Butyl-dimethyl-silanyloxy)-5-(4-fluor-phenyl)-1-(4-methoxy-phenyl)-2-(2-oxo-4-phenyl-oxazolidin-3-carbonyl)-pentylamino]-benzonitril werden in 380 ml Methyl-tert.-Butylether gelöst, mit 18,6 ml N,O-Bis(trimethylsilyl)-acetamid und 1,86 ml einer 1 M Lösung von Tetrabutylammoniumfluorid in Tetrahydrofuran versetzt und 2 h bei Raumtemperatur gerührt. Nach Beendigung der Reaktion fügt man 10 ml Essigsäure zu, engt die Reaktionsmischung im Vakuum ein und reinigt den Rückstand über Kieselgel mit Toluol/Essigsäureethylester 50/1. Man erhält 4-[3-[3-(tert-Butyl-dimethyl-silanyloxy)-3-(4-fluor-phenyl)-propyl]-2-(4-methoxy-phenyl)-4-oxo-azetidin-1-yl]-benzonitril mit dem Molekulargewicht 544,75 (C₃₂H₃₇FN₂O₃Si); MS (ESI): 545.56 (M+H⁺).

### f) 4-[3-[3-(4-Fluor-phenyl)-3-hydroxy-propyl]-2-(4-methoxy-phenyl)-4-oxo-azetidin-1-yl]- benzonitril (41)

3.5 g 4-[3-[3-(tert-Butyl-dimethyl-silanyloxy)-3-(4-fluor-phenyl)-propyl]-2-(4-methoxyphenyl)-4-oxo-azetidin-1-yl]-benzonitril werden in 65 ml Tetrahydrofuran gelöst, mit 0,74 ml Essigsäure und 8,03 ml einer 1 M Lösung von Tetrabutylammoniumfluorid in Tetrahydrofuran versetzt und 2 h bei Raumtemperatur gerührt. Danach werden 4,82 ml der Tetrabutylammoniumfluorid-Lösung nachgegeben und weitere 3 h bei Rückflusstemperatur gerührt. Die abgekühlte Reaktionsmischung wird im Vakuum eingeengt, und der Rückstand wird chromatographisch über Kieselgel mit n-Heptan/Essigsäureethylester 2/1 gereinigt. Man erhält 4-[3-[3-(4-Fluor-phenyl)-3-hydroxy-propyl]-2-(4-methoxy-phenyl)-4-oxo-azetidin-1-yl]- benzonitril mit dem Molekulargewicht 430,48 (C₂₆H₂₃FN₂O₃); MS (ESI): 431.24 (M+H⁺).

### g) 1-(4-Aminomethyl-phenyl)-3-[3-(4-fluor-phenyl)-3-hydroxy-propyl]-4-(4-methoxyphenyl)- azetidin-2-on (42)

1,22 g 4-[3-[3-(4-Fluor-phenyl)-3-hydroxy-propyl]-2-(4-methoxy-phenyl)-4-oxo-azetidin-1-yl]- benzonitril werden in 90 ml Ethanol gelöst, mit 10 ml konz. Ammoniaklösung und einem Überschuß Raney-Nickel versetzt und 8 h bei 60°C und einem Druck von 10 bar Wasserstoff gerührt. Die Reaktionsmischung kühlt über Nacht auf Raumtemperatur ab; anderntags wird vom Katalysator abgetrennt, das Filtrat im Vakuum eingeengt und der Rückstand chromatographisch über Kieselgel mit Dichlormethan/Methanol/Ammoniak-Lösung 10/1/0.1 gereinigt. Man erhält 1-(4-Aminomethyl-phenyl)-3-[3-(4-fluor-phenyl)-3-hydroxy-propyl]-4-(4-methoxy-phenyl)-azetidin-2-on mit dem Molekulargewicht 434,51 (C₂₆H₂₇FN₂O₃); MS (ESI): 418.2 (MH⁺-NH₃).

### h) Benzyl-(4-{4-[3-[3-(4-fluor-phenyl)-3-hydroxy-propyl]-2-(4-methoxy-phenyl)-4-oxo-azetidin-1-yl]-benzylcarbamoyl}-butyl)-(2,3,4,5,6-pentahydroxy-hexyl)-ammonium; trifluoracetat (35)

100 mg 5-[Benzyl-(2,3,4,5,6-pentahydroxy-hexyl)-amino]-pentansäure und 110 mg 1-(4-Aminomethyl-phenyl)-3-[3-(4-fluor-phenyl)-3-hydroxy-propyl]-4-(4-methoxyphenyl)-azetidin-2-on werden bei Raumtemperatur in 2 ml trockenem Dimethylformamid gelöst, mit 42 mg N-Hydroxy-Benzotriazol und 52 mg 1-Ethyl-3-(3-dimethylaminopropyl)carbodiimid Hydrochlorid versetzt und über Nacht bei Raumtemperatur gerührt. Anderntags wird die Reaktionsmischung im Vakuum eingeengt und zur Reinigung über RP18 mit Acetonitril/Wasser mit 0,1% Trifluoressigsäure chromatographiert. Man erhält Benzyl-(4-{4-[3-[3-(4-fluor-phenyl)-3-hydroxy-propyl]-2-(4-methoxy-phenyl)-4-oxo-azetidin-1-yl]-benzylcarbamoyl}-butyl)-(2,3,4,5,6-pentahydroxy-hexyl)-ammonium; trifluoracetat mit dem Molekulargewicht 787,93 (C₄₄H₅₄FN₃O₉; Kation); MS (ESI): 788.70 (M+H⁺).

### Beispiel XIX

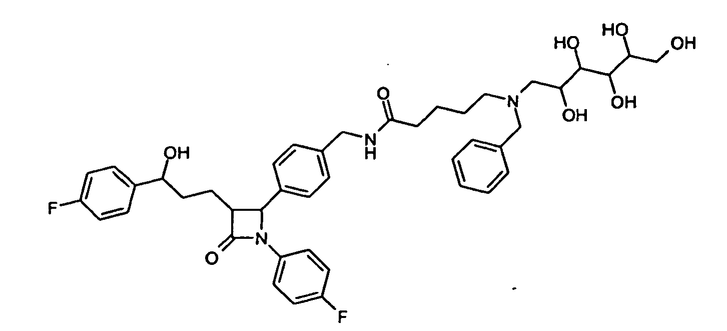

### 5-[Benzyl-(2,3,4,5,6-pentahydroxy-hexyl)-amino]-pentansäure 4-{1-(4-fluor-phenyl)-3- [3-(4-fluor-phenyl)-3-hydroxy-propyl]-4-oxo-azetidin-2-yl}-benzylamid (43)

Die Verbindung des Beispiels XIX wird ausgehend von 5-[Benzyl-(2,3,4,5,6-pentahydroxy-hexyl)-amino]-pentansäure und 4-(4-Aminomethyl-phenyl)-1-(4-fluorphenyl)-3-[3-(4-fluor-phenyl)-3-hydroxy-propyl]- azetidin-2-on analog der Verbindung des Beispiels XVIII hergestellt. Man erhält 5-[Benzyl-(2,3,4,5,6-pentahydroxy-hexyl)-amino]-pentansäure 4-{1-(4-fluor-phenyl)-3- [3-(4-fluor-phenyl)-3-hydroxy-propyl]-4-oxo-azetidin-2-yl}-benzylamid mit dem Molekulargewicht 775,89 (C₄₃H₅₁F₂N₃O₈); MS (ESI): 776.4 (M+H⁺).

### 1-(4-Fluor-phenyl)-3-[3-(4-fluor-phenyl)-3-hydroxy-propyl]-4-[4-(4-iod-butoxy)-phenyl]-azetidin-2-on (55)

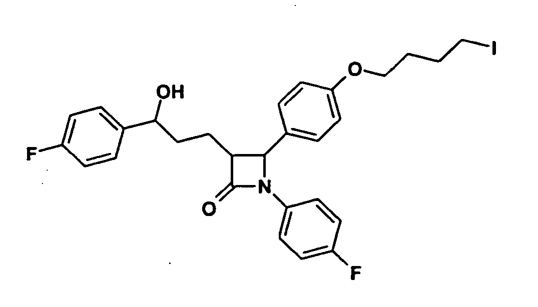

In 10 ml absolutem Dimethylformamid werden 100 mg 1-(4-Fluor-phenyl)-3-[3-(4-fluor-phenyl)-3-hydroxy-propyl]-4-(4-hydroxy-phenyl)-azetidin-2-on gelöst. Dann werden 80 mg gepulvertes Kaliumcarbonat und 0,2 ml Diiodbutan zugegeben. Die Reaktionslösung wird über Nacht bei Raumtemperatur gerührt. Nach dem Einengen am Rotationsverdampfer unter Ölpumpenvakuum bei 40 °C wird der Rückstand über eine SiO₂-Kartusche (n-Heptan; n-Heptan / Ethylacetat 4 : 1) gereinigt. Das Produkt wird als Öl erhalten. C₂₈H₂₈F₂INO₃ (591) MS (ESI): M⁺-18

### Beispiel XXV

### 1-(4-Fluor-phenyl)-3-[3-(4-fluor-phenyl)-3-hydroxy-propyl]-4-(4-{4-[methyl-(2,3,4,5,6-pentahydroxy-hexyl)-amino]-butoxy}-phenyl)-azetidin-2-on (56)

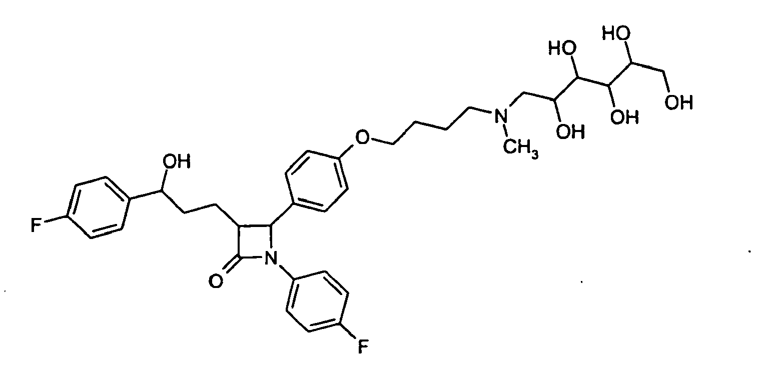

In 5 ml absolutem Dimethylformamid werden 100 mg 1-(4-Fluor-phenyl)-3-[3-(4-fluorphenyl)-3-hydroxy-propyl]-4-[4-(4-iod-butoxy)-phenyl]-azetidin-2-on gelöst. Dann werden 132 mg 6-Methylarriino-hexan-1,2,3,4,5-pentanol zugegeben und die Reaktionslösung wird bei 50 °C 2 h lang gerührt. Nach dem Einengen am Rotationsverdampfer unter Ölpumpenvakuum bei 40 °C wird der Rückstand über präparative HPLC gereinigt. Das Produkt (89 mg) wird als Öl erhalten. C₃₅H₄₄F₂N₂O₈ (658) MS (ESI): M⁺

### 1-(4-Fluor-phenyl)-3-[3-(4-fluor-phenyl)-3-hydroxy-propyl]-4-[4-(5-iod-pentyloxy)-phenyl]-azetidin-2-on (57)

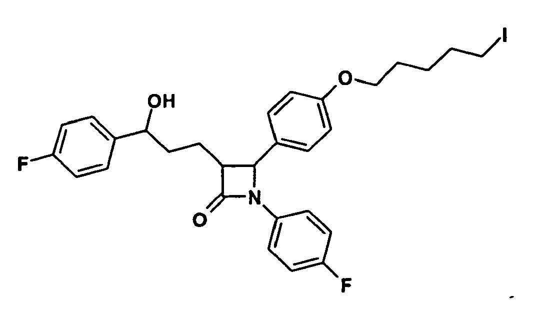

In 10 ml absolutem Dimethylformamid werden 150 mg 1-(4-Fluor-phenyl)-3-[3-(4-fluor-phenyl)-3-hydroxy-propyl]-4-(4-hydroxy-phenyl)-azetidin-2-on gelöst. Dann werden 120 mg gepulvertes Kaliumcarbonat und 0,33 ml Diiodpentan zugegeben. Die Reaktionslösung wird über Nacht bei Raumtemperatur gerührt. Nach dem Einengen am Rotationsverdampfer unter Ölpumpenvakuum bei 40 °C wird der Rückstand über eine SiO₂-Kartusche (n-Heptan; n-Heptan / Ethylacetat 4 : 1) gereinigt. Das Produkt wird als Öl erhalten. C₂₉H₃₀F₂INO₃ (605) MS (ESI): M⁺-18

### Beispiel XXVI

### 1-(4-Fluor-phenyl)-3-[3-(4-fluor-phenyl)-3-hydroxy-propyl]-4-(4-{5-[methyl-(2,3,4,5,6-pentahydroxy-hexyl)-amino]-pentyloxy}-phenyl)-azetidin-2-on (58)

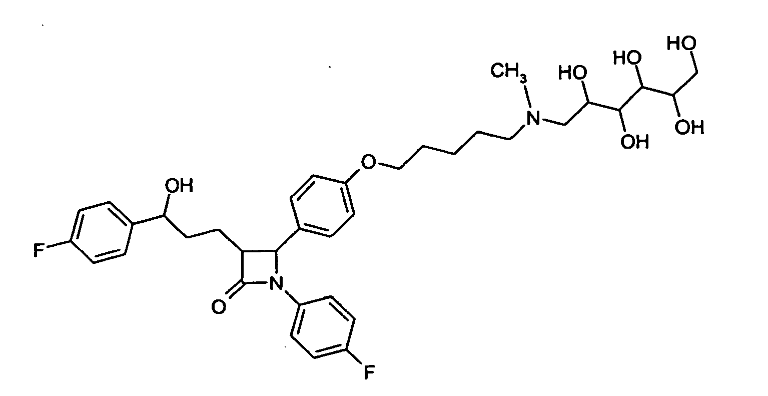

In 5 ml absolutem Dimethylformamid werden 170 mg 1-(4-Fluor-phenyl)-3-[3-(4-fluor-pheny))-3-hydroxy-propy)]-4-[4-(5-iod-penty)oxy)- phenyl]-azetidin-2-on gelöst. Dann werden 220 mg 6-Methylamino-hexan-1,2,3,4,5-pentanol zugegeben und die Reaktionslösung wird bei 50°C 2 h lang gerührt. Nach dem Einengen am Rotationsverdampfer unter Ölpumpenvakuum bei 40 °C wird der Rückstand über präparative HPLC gereinigt. Das Produkt wird als Öl erhalten. C₃₆H₄₆F₂N₂O₈ (672) MS (ESI): M⁺

### 1-(4-Fluor-phenyl)-3-[3-(4-fluor-phenyl)-3-hydroxy-propyl]-4-[4-(6-iod-hexyloxy)-phenyl]-azetidin-2-on (59)

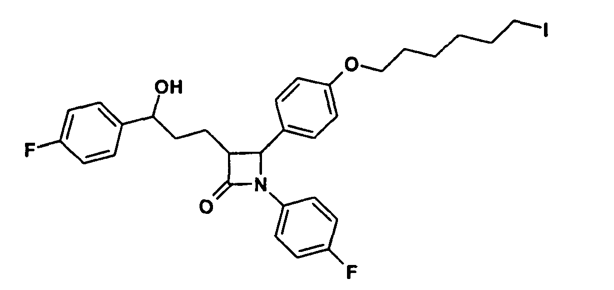

In 10 ml absolutem Dimethylformamid werden 100 mg 1-(4-Fluor-phenyl)-3-[3-(4-fluor-phenyl)-3-hydroxy-propyl]-4-(4-hydroxy-phenyl)-azetidin-2-on gelöst. Dann werden 80 mg gepulvertes Kaliumcarbonat und 0,25 ml Diiodhexan zugegeben. Die Reaktionslösung wird über Nacht bei Raumtemperatur gerührt. Nach dem Einengen am Rotationsverdampfer unter Ölpumpenvakuum bei 40 °C wird der Rückstand über eine SiO₂-Kartusche (n-Heptan; n-Heptan / Ethylacetat 4 : 1) gereinigt. Das Produkt wird als Öl erhalten. C₃₀H₃₂F₂lNO₃ (619) MS (ESI): M⁺-18

### Beispiel XXVII

### 1-(4-Fluor-phenyl)-3-[3-(4-fluor-phenyl)-3-hydroxy-propyl]-4-(4-{6-[methyl-(2,3,4,5,6-pentahydroxy-hexyl)-amino]-hexyloxy}-phenyl)-azetidin-2-on (60)

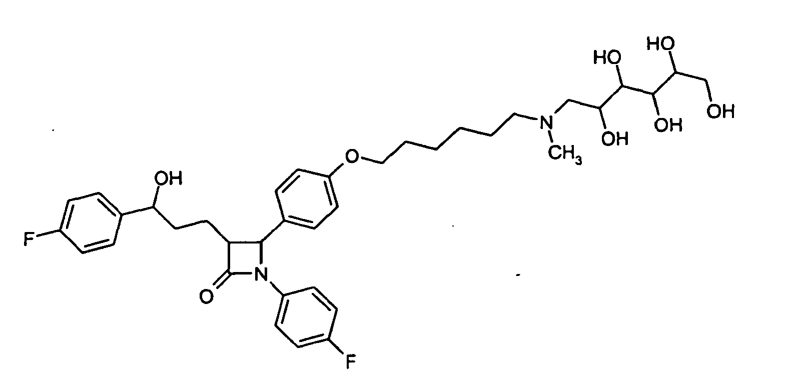

In 10 ml absolutem Dimethylformamid werden 136 mg 1-(4-Fluor-phenyl)-3-[3-(4-fluor-phenyl)-3-hydroxy-propyl]-4-[4-(6-iod-hexyloxy)-phenyl]-azetidin-2-on gelöst. Dann werden 172 mg 6-Methylamino-hexan-1,2,3,4,5-pentanol zugegeben und die Reaktionslösung wird bei 50 °C 2,5 h lang gerührt. Nach dem Einengen am Rotationsverdampfer unter Ölpumpenvakuum bei 40 °C wird der Rückstand über präparative HPLC gereinigt. Das Produkt wird als Öl erhalten. C₃₇H₄₈F₂N₂O₈ (686) MS (ESI): M⁺

### 1-(4-Fluor-phenyl)-3-[3-(4-fluor-phenyl)-3-hydroxy-propyl]-4-[4-(8-iod-octyloxy)-phenyl]-azetidin-2-on (61)

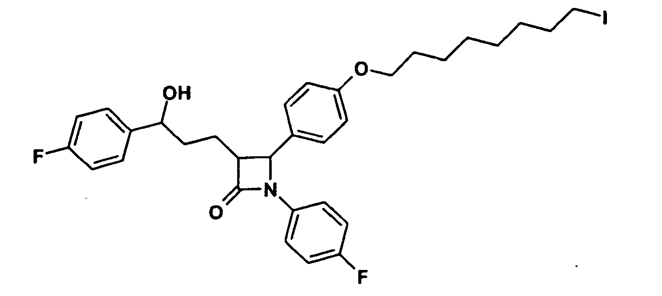

In 10 ml absolutem Dimethylformamid werden 150 mg 1-(4-Fluor-phenyl)-3-[3-(4-fluor-phenyl)-3-hydroxy-propyl]-4-(4-hydroxy-phenyl)-azetidin-2-on gelöst. Dann werden 120 mg gepulvertes Kaliumcarbonat und 0,44 ml Diiodoctan zugegeben. Die Reaktionslösung wird über Nacht bei Raumtemperatur gerührt. Nach dem Einengen am Rotationsverdampfer unter Ölpumpenvakuum bei 40 °C wird der Rückstand über eine SiO₂-Kartusche (n-Heptan; n-Heptan / Ethylacetat 4 : 1) gereinigt. Das Produkt wird als Öl erhalten. C₃₂H₃₆F₂INO₃ (647) MS (ESI): M⁺-18

### Beispiel XXVIII

### 1-(4-Fluor-phenyl)-3-[3-(4-fluor-phenyl)-3-hydroxy-propyl]-4-(4-{8-[methyl-(2,3,4,5,6-pentahydroxy-hexyl)-amino]-octyloxy}-phenyl)-azetidin-2-on (62)

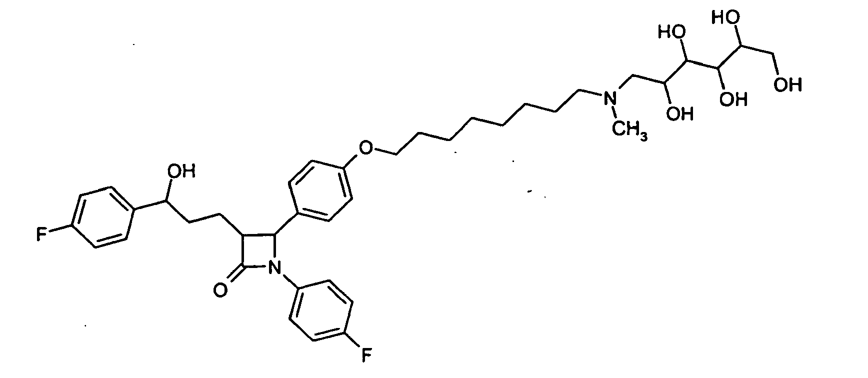

In 5 ml absolutem Dimethylformamid werden 150 mg 1-(4-Fluor-phenyl)-3-[3-(4-fluorphenyl)-3-hydroxy-propyl]-4-[4-(8-iod-octyloxy)-phenyl]-azetidin-2-on gelöst. Dann werden 180 mg 6-Methylamino-hexan-1,2,3,4,5-pentanol zugegeben und die Reaktionslösung wird bei 50 °C 2 h lang gerührt. Nach dem Einengen am Rotationsverdampfer unter Ölpumpenvakuum bei 40 °C wird der Rückstand über präparative HPLC gereinigt. Das Produkt wird als Öl erhalten. C₃₉H₅₂F₂N₂O₈ (714) MS (ESI): M⁺

### 1-(4-Fluor-phenyl)-3-[3-(4-fluor-phenyl)-3-hydroxy-propyl]-4-[4-(10-iod-decyloxy)-phenyl]-azetidin-2-on (63)

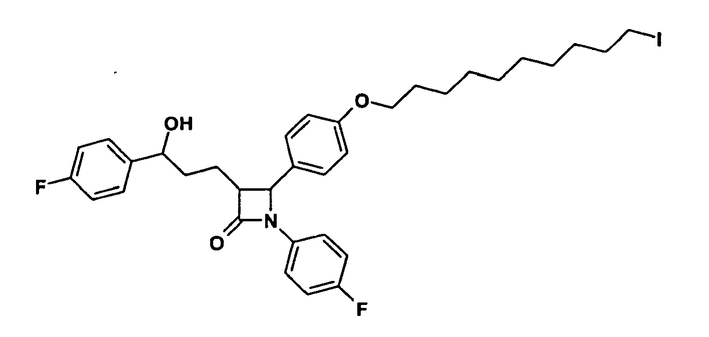

In 10 ml absolutem Dimethylformamid werden 150 mg 1-(4-Fluor-phenyl)-3-[3-(4-fluor-phenyl)-3-hydroxy-propyl]-4-(4-hydroxy-phenyl)-azetidin-2-on gelöst. Dann werden 120 mg gepulvertes Kaliumcarbonat und 865 mg Diioddecan zugegeben. Die Reaktionslösung wird über Nacht bei Raumtemperatur gerührt. Nach dem Einengen am Rotationsverdampfer unter Ölpumpenvakuum bei 40 °C wird der Rückstand über eine SiO₂-Kartusche (n-Heptan; n-Heptan / Ethylacetat 4 : 1) gereinigt. Das Produkt wird als Öl erhalten. C₃₄H₄₀F₂INO₃ (675) MS (ESI): M⁺-18

### Beispiel XXIX

### 1-(4-Fluor-phenyl)-3-[3-(4-fluor-phenyl)-3-hydroxy-propyl]-4-(4-{10-[methyl-(2,3,4,5,6-pentahydroxy-hexyl)-amino]-decyloxy}-phenyl)-azetidin-2-on (64)

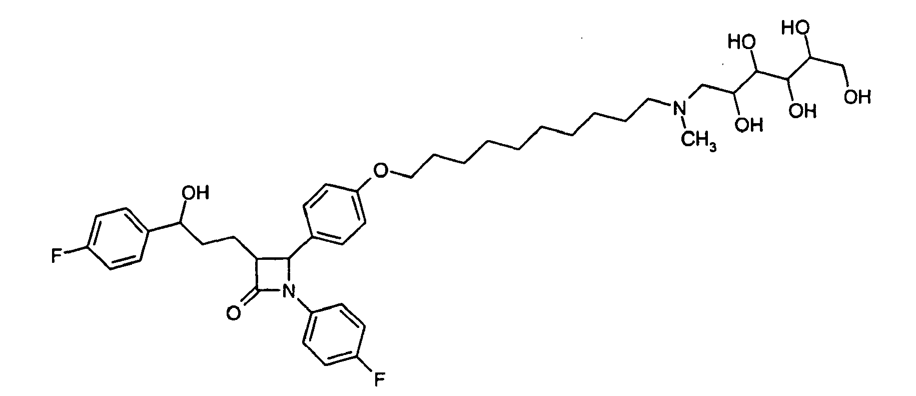

In 5 ml absolutem Dimethylformamid werden 170 mg 1-(4-Fluor-phenyl)-3-[3-(4-fluorphenyl)-3-hydroxy-propyl]-4-[4-(10-iod-decyloxy)-phenyl]-azetidin-2-on gelöst. Dann werden 200 mg 6-Methylamino-hexan-1,2,3,4,5-pentanol zugegeben und die Reaktionslösung wird bei 50 °C 2 h lang gerührt. Nach dem Einengen am Rotationsverdampfer unter Ölpumpenvakuum bei 40 °C wird der Rückstand über präparative HPLC gereinigt. Das Produkt wird als Öl erhalten. C₄₁H₅₆F₂N₂O₈ (742) MS (ESI): M⁺

### 1-(4-Fluor-phenyl)-3-[3-(4-fluor-phenyl)-3-hydroxy-propyl]-4-(4-{2-[2-(2-iod-ethoxy)-ethoxy]-ethoxy}-phenyl)-azetidin-2-on (65)

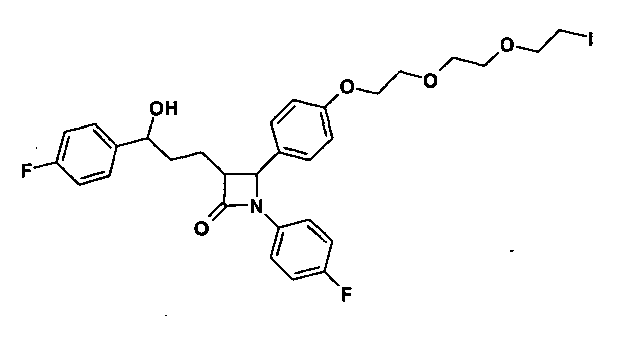

In 10 ml absolutem Dimethylformamid werden 150 mg 1-(4-Fluor-phenyl)-3-[3-(4-fluor-phenyl)-3-hydroxy-propyl]-4-(4-hydroxy-phenyl)-azetidin-2-on gelöst. Dann werden 120 mg gepulvertes Kaliumcarbonat und 0,4 ml 1,2-bis(Diiodethoxy)ethan zugegeben. Die Reaktionslösung wird über Nacht bei Raumtemperatur gerührt.

Nach dem Einengen am Rotationsverdampfer unter Ölpumpenvakuum bei 40 °C wird der Rückstand über eine SiO₂-Kartusche (n-Heptan; n-Heptan / Ethylacetat 4 : 1) gereinigt. Das Produkt wird als Öl erhalten. C₃₀H₃₂F₂INO₅ (651) MS (ESI): M⁺-18

### Beispiel XXX

### 1-(4-Fluor-phenyl)-3-[3-(4-fluor-phenyl)-3-hydroxy-propyl]-4-{4-[2-(2-{2-[methyl-(2,3,4,5,6-pentahydroxy-hexyl)-amino]-ethoxy}-ethoxy)-ethoxy]-phenyl}-azetidin-2-on (66)

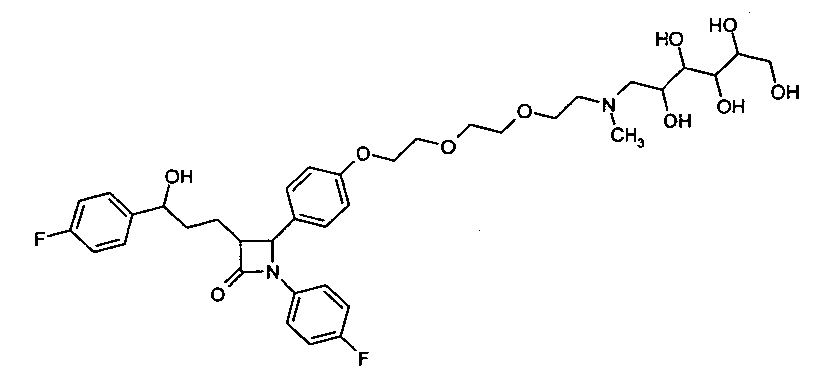

In 5 ml absolutem Dimethylformamid werden 230 mg 1-(4-Fluor-phenyl)-3-[3-(4-fluor-phenyl)-3-hydroxy-propyl]-4-(4-{2-[2-(2-iod-ethoxy)-ethoxy]-ethoxy}-phenyl)-azetidin-2-on gelöst. Dann werden 280 mg 6-Methylamino-hexan-1,2,3,4,5-pentanol zugegeben und die Reaktionslösung wird bei 50 °C 2 h lang gerührt. Nach dem Einengen am Rotationsverdampfer unter Ölpumpenvakuum bei 40°C wird der Rückstand über präparative HPLC gereinigt. Das Produkt wird als Öl erhalten. C₃₇H₄₈F₂N₂O₁₀ (718) MS (ESI): M⁺

### Hex-5-ensäure-methyl-(2,3,4,5,6-pentahydroxy-hexyl)-amid (67)

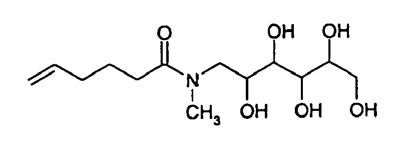

In 3 ml absolutem Methylendichlorid werden 1,11g 5-Hexensäure gelöst. Dann werden 1,4 ml Thionylchlorid zugetropft. Bei Raumtemperatur wird 3 h lang gerührt und danach am Rotationsverdampfer eingeengt. In 5 ml absolutem Methylendichlorid werden 1,09 g 6-Methylamino-hexan-1,2,3,4,5-pentanol suspendiert. Nach Zutropfen von in 3 ml absolutem Methylendichlorid gelöstem 5-Hexensäurechlorid wird 4 h lang bei Raumtemperatur gerührt. Der entstandene Niederschlag wird vom Reaktionsprodukt abfiltriert, das Filtrat am Rotationsverdampfer eingeengt und das ölige Rohprodukt ohne Reinigung weiter umgesetzt. C₁₃H₂₅NO₆ (291) MS (ESI): M⁺

### 6-{4-[3-[3-(tert-Butyl-dimethyl-silanyloxy)-3-(4-fluor-phenyl)-propyl]-1-(4-fluor-phenyl)-4-oxo-azetidin-2-yl]-phenyl}-hex-5-ensäure-methyl-(2,3,4,5,6-pentahydroxy-hexyl)-amid (68)

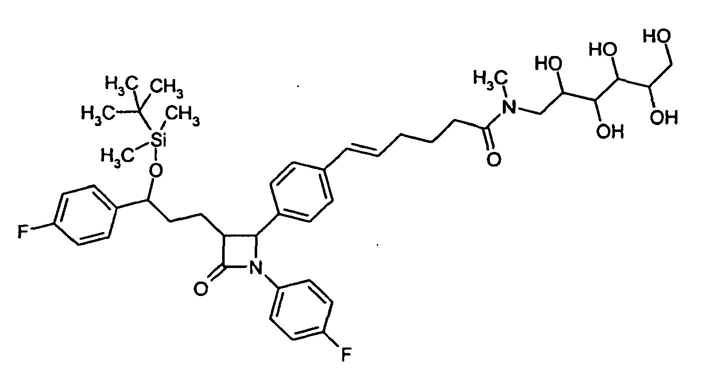

In 300 µl Triethylamin werden 110 mg 4-(4-Brom-phenyl)-3-[3-(tert-butyl-dimethyl-silanyloxy)-3-(4-fluor-phenyl)-propyl]-1-(4-fluor-phenyl)-azetidin-2-on und 136 mg Hex-5-ensäure-methyl-(2,3,4,5,6-pentahydroxy-hexyl)-amid in einem ausgeglühten, geschlossenen Röhrchen unter Argon vorgelegt. Nach Zugabe von 6 mg Palladiumacetat und 14 mg Triphenylphosphin wird bei 100 °C 4 h lang gerührt. Die Reaktionsmischung wird dann in Dichlormethan aufgenommen, filtriert und am Rotationsverdampfer eingeengt. Reinigung des Rückstandes über eine SiO₂-Kartusche (Dichlormethan / Methanol 20 : 1 - 5 : 1) ergibt das Produkt. C₄₃H₅₈F₂N₂O₈Si (796)

### Beispiel XXXI

### 6-(4-{1-(4-Fluor-phenyl)-3-[3-(4-fluor-phenyl)-3-hydroxy-propyl]-4-oxo-azetidin-2-yl}-phenyl)-hex-5-ensäure-methyl-(2,3,4,5,6-pentahydroxy-hexyl)-amid (69)

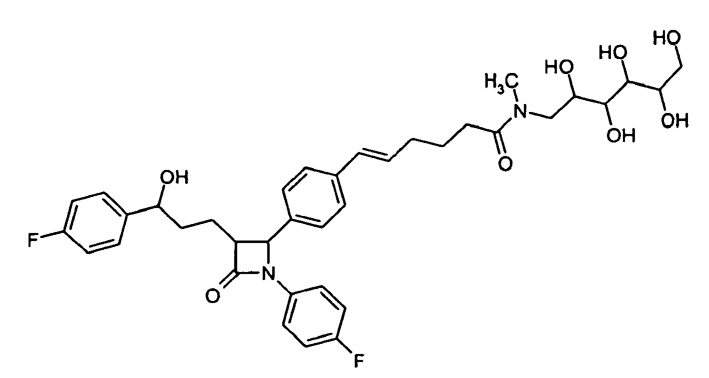

In 6 ml Methanol werden 70 mg 6-{4-[3-[3-(tert-Butyl-dimethyl-silanyloxy)-3-(4-fluorphenyl)-propyl]-1-(4-fluor-phenyl)-4-oxo-azetidin-2-yl]-phenyl}-hex-5-ensäure-methyl-(2,3,4,5,6-pentahydroxy-hexyl)-amid gelöst. Dann werden 0,1 N HCl_{(aq)} zugegeben und bei Raumtemperatur über Nacht gerührt. Danach wird mit 1 N Natronlauge neutralisiert und am Rotationsverdampfer eingeengt. Der Rückstand wird mit Dichlormethan verrührt, filtriert und die Mutterlauge am Rotationsverdampfer eingeengt. Man erhält das Produkt nach Reinigung über präparative HPLC: C₃₁H₄₄F₂N₂O₈ (682) MS (ESI): M⁺-18

### Beispiel XXXV

### Essigsäure-2,3,4,5-tetraacetoxy-1-{3-[3-[3-(4-fluor-phenyl)-3-hydroxy-propyl]-2-(4-methoxy-phenyl)-4-oxo-azetidin-1-yl]-benzylcarbamoyl}-pentyl ester (73)

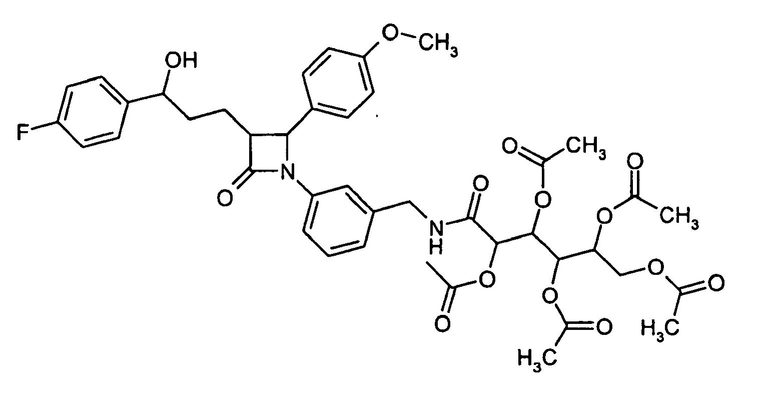

112 mg (0.24 mmol) 1-(3-Aminomethyl-phenyl)-3-[3-(4-fluor-phenyl)-3-hydroxypropyl]-4-(4-methoxy-phenyl)- azetidin-2-on werden in 5 ml Methylenchlorid und 0.5 ml Triethylamin gelöst. Bei 0 °C gibt man 0.5 g Essigsäure-2,3,4-triacetoxy-1-(acetoxy-chlorocarbonyl-methyl)-butylester zu und läßt auf Raumtemperatur auftauen. Nach 30 Minuten wird mit Ethylacetat verdünnt und dann über Kieselgel filtriert. Das Lösungsmittel wird abdestilliert und der Rückstand mit Flashchromatochraphie gereinigt. Man erhält das Produkt als amorphen Feststoff: C₄₂H₄₇FN₂O₁₄ (822.84) MS (ESI): M⁺= 823.3.

### Beispiel XXXVI

### 2,3,4,5,6-Pentahydroxy-hexansäure 3-[3-[3-(4-fluor-phenyl)-3-hydroxy-propyl]-2-(4-methoxy-phenyl)-4-oxo-azetidin-1-yl]-benzylamid (74)

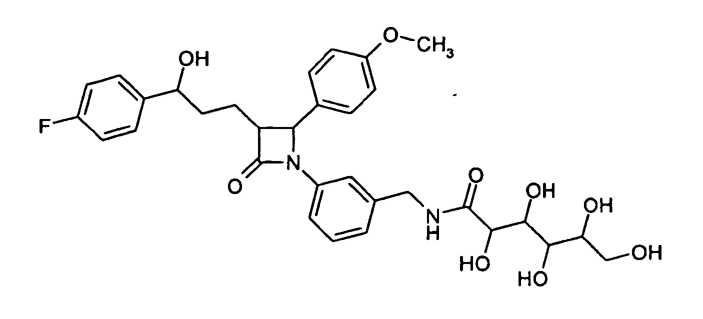

90 mg (109 µmol) Essigsäure-2,3,4,5-tetraacetoxy-1-{3-[3-[3-(4-fluoro-phenyl)-3-hydroxy-propyl]-2-(4- methoxy-phenyl)-4-oxo-azetidin-1-yl]-benzylcarbamoyl}-pentyl ester werden in 7 ml Methanol gelöst und mit 0.5 ml 1 N NaOMe/MeOH versetzt. Nach 2 Stunden bei Raumtemperatur wird mit methanolischer Salzsäure neutralisiert und eingeengt. Der Rückstand wird mit Flashchromatographie gereinigt. Das Produkt erhält man als amorphen Feststoff. C₃₂H₃₇FN₂O₉ (612.66) MS (ESI): M⁺= 613.2.

### Beispiel XXXVII

### 6-(4-{3-[1-(4-Fluor-phenyl)-2-(4-methoxy-phenyl)-4-oxo-azetidin-3-yl]-1-hydroxy-propyl}-phenyl)-hex-5-ensäure methyl-(2,3,4,5,6-pentahydroxy-hexyl)-amid (75)

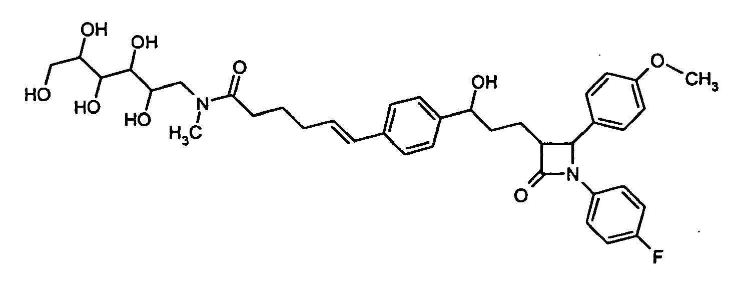

200 mg Hex-5-ensäure methyl-(2,3,4,5,6-pentahydroxy-hexyl)-amid und 72 mg 3-[3-(4-Brom-phenyl)-3-hydroxy-propyl]-1-(4-fluor-phenyl)-4-(4-methoxy-phenyl)-azetidin-2-on werden analog der Synthese von Beispiel XXXI hergestellt. Man erhält das Produkt als amorphen Feststoff.

Die erfindungsgemäßen Verbindungen der Formel I wurden mit der nachfolgend beschriebenen Methode auf ihre Wirkung geprüft:

### Beeinflussung der Cholesterolabsorption + ³H- Taurocholsäureausscheidung anhand der fäkalen Ausscheidung an der Maus, Ratte oder Hamster

NMRI- Mäuse, Wistar-Ratten, oder Golden Syrian Hamster (in Gruppen von n=4-6) werden unter Standarddiät (Altromin, Lage (Lippe)) in Stoffwechselkäfigen gehalten. Am Nachmittag vor Gabe der radioaktiven Tracer (¹⁴C-Cholesterol) werden die Tiere nüchtern gesetzt und auf Gitterroste adaptiert.

Zusätzlich werden die Tiere werden 24 Stunden vor der peroralen Applikation der Testmahlzeit (¹⁴C-Cholesterol in Intralipid® 20, Pharmacia-Upjohn) mit ³H-TCA (Taurocholic acid) s.c. gelabelt (z.b. 1 µCi/Maus bis 5 µCi/Ratte)

Cholesterolabsorptionstest: 0,25 mI/Maus Intralipid ® 20 (Pharmacia- Upjohn) ((Spikung mit 0,25 µCi ¹⁴C-Cholesterol in 0,1 mg Cholesterol) werden peroral mit der Schlundsonde verabreicht.

Testsubstanzen werden getrennt in 0,5 %/ (Methylcellulose (Sigma)/5% Solutol (BASF, Ludwigshafen) oder geeignetem Vehikel angesetzt.
Das Applikationsvolumen der Testsubstanz beträgt 0,5 ml/Maus. Die Testsubstanz wird unmittelbar vor der Testmahlzeit (Intralipid mit ¹⁴C-Cholesterol-label) (Cholesterolabsorptionstest) appliziert.

Der Kot wird über 24 h gesammelt: die fäkale Elimination von ¹⁴C-Cholesterol und ³H Taurocholsäure (TCA) nach 24 Std. wird bestimmt.

Die Lebern werden entnommen, homogenisiert und Aliquots im Oximaten (Model 307, Packard) verbrannt zur Bestimmung der aufgenommenn/resorbierten Menge an ¹⁴C- Cholesterol.

### Auswertung:

### Kotproben:

Gesamtgewicht bestimmen, mit Wasser auf definiertes Volumen auffüllen, dann homogenisieren, Aliquot eintrockenen und im Oximat (Model 307, Packard zur Verbrennung von radioaktiv gelabelten Proben) verbrennen: Die Menge von radioaktiv ³H- H2O und ¹⁴C- CO2 wird hochgerechnet auf die ausgeschiedene Menge an ³H-Taurocholsäure bzw. ¹⁴C-Cholesterol (Dual-Isotopen-Technik). Die ED₂₀₀-Werte werden als Dosis aus einer Dosiswirkungskurve interpoliert als diejenige Dosen, die die Auscheidung an TCA bzw. Cholesterol verdoppeln, bezogen auf eine zeitgleich behandelte Kontrollgruppe.

### Leberproben:

Die aufgenommene Menge von ¹⁴C-Cholesterols in die Leber wird bezogen auf die applizierte Dosis. Die ED₅₀ Werte werden interpoliert aus einer Dosiswirkungskurve als diejenige Dosis, die die Aufnahme von ¹⁴C- Cholesterol in die Leber halbiert (50%), bezogen auf eine Kontrollgruppe

Die folgenden ED₅₀-Werte belegen die Aktivität der erfindungsgemäßen Verbindungen der Formel I

| Beispiel Nr. | ED₅₀ (Leber) [mg/Maus] |
|---|---|
| II | 0.1 |
| III | 0.003 |
| XIII | 0.3 |
| XVIII | 1.0 |
| XXV | 0.3 |
| XXX | 0.1 |

Aus der Tabelle ist abzulesen, daß die Verbindungen der Formel I eine sehr gute Cholesterin senkende Wirkung besitzen.

### Resorbierbarkeit:

Die Resorbierbarkeit der Verbindungen der Formel I wurde Caco-Zellmodell geprüft (A.R. Hilgers et al., Caco-2 cell monolayers as a model for drug transport across the intestinal mucosa, Pharm. Res. 1990, 7, 902).

Aus den Meßdaten ist abzulesen, daß die erfindungsgemäßen Verbindungen der Formel I gegenüber den im Stand der Technik beschriebenen Verbindungen (Referenzstruktur) eine deutlich geringere Resorption aufweisen:

| | Referenzstruktur | Beispiel |
|---|---|---|
| Apparenter Partitionskoeffizient Pₐₚₚ [cm/s] | 4.88 x 10⁻⁰⁶ | |
| (entsprechend Lit. Hilgers) | | |
| Abgeschätzte Human-Resorption 100% | | |

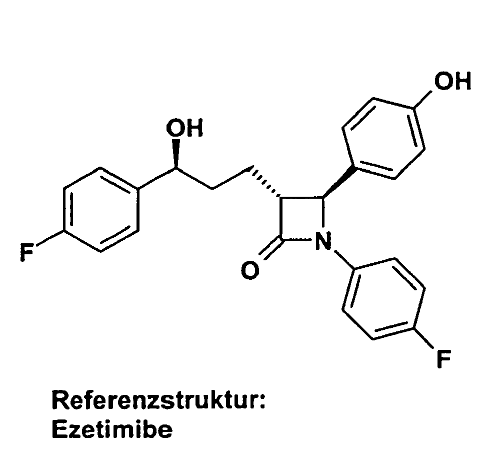

## Patentansprüche

1. Verbindungen der Formel I, 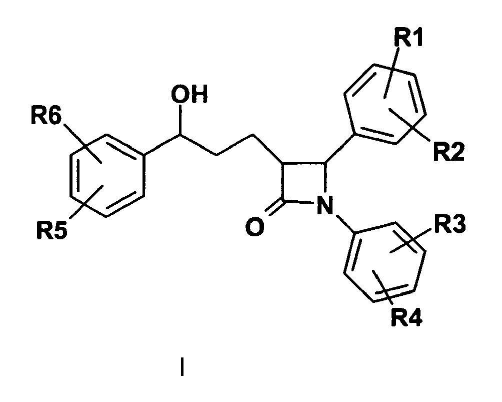 worin bedeuten
R1, R3 unabhängig voneinander -(CH₂)₀₋₁-NH-(C=O)₀₋₁-(C₀-C₂₅)-Alkylen-(C=O)₀₋₁-N(R7)₀₋₁-LAG, wobei ein oder mehrere C-Atome des Alkylenrests durch O-Atome ersetzt sein können,
H, F, Cl, Br, I, CF₃, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
SO₂-NH₂, SO₂NH(C₁-C₆)-Alkyl, SO₂N[(C₁-C₆)-Alkyl]₂, S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, CI, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
R2, R4, R5, R6 unabhängig voneinander -(CH₂)₀₋₁-NH-(C=O)₀₋₁-(C₃-C₂₅)-Alkylen-(C=O)₀₋₁-N(R7)₀₋₁-LAG ,wobei ein oder mehrere C-Atome des Alkylenrests durch O-Atome ersetzt sein können,
H, F, Cl, Br, I, CF₃, NO₂, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können;
SO₂-NH₂, SO₂NH(C₁-C₆)-Alkyl, SO₂N[(C₁-C₆)-Alkyl]₂, S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
R7 H, CH₃;
(LAG) Zuckerrest;
worin einer der Reste R1 oder R3 die Bedeutung -(CH₂)₀₋₁-NH-(C=O)₀₋₁-(C₀-C₂₅)-Alkylen-(C=O)₀₋₁-N(R7)₀₋₁-LAG ,wobei ein oder mehrere C-Atome des Alkylenrests durch O-Atome ersetzt sein können, besitzt, sowie deren pharmazeutisch verträglichen Salze.

2. Arzneimittel enthaltend eine oder mehrere der Verbindungen gemäß Anspruch 1.

3. Arzneimittel enthaltend eine oder mehrere der Verbindungen gemäß Anspruch 1 und mindestens einen weiteren Wirkstoff.

4. Arzneimittel, gemäß Anspruch 3, **dadurch gekennzeichnet, daß** es als weiteren Wirkstoff eine oder mehrere Verbindungen, die den Lipidstoffwechsel normalisieren, enthält.

5. Arzneimittel, gemäß Anspruch 3 oder 4, **dadurch gekennzeichnet, daß** es als weiteren Wirkstoff eine oder mehrere
Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, Fibrate, MTP-Inhibitoren, Gallensäureresorptionsinhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen synthetase inhibitoren, Lipoprotein(a) antagonisten, Lipase Inhibitoren, Insuline, Sulphonylharnstoffe, Biguanide, Meglitinide, Thiazolidindione, α-Glukosidase-Inhibitoren, auf den ATP-abhängigen Kaliumkanal der Betazellen wirkende Wirkstoffe, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten oder Amphetamine enthält.

6. Arzneimittel, gemäß Anspruch 5, **dadurch gekennzeichnet, daß** es als HMGCoA-Reduktase Inhibitor Simvastatin, Fluvastatin, Pravastatin, Lovastatin, Atorvastatin, Cerivastatin oder Rosuvastatin enthält.

7. Verbindungen gemäß Anspruch 1 zur Anwendung als Medikament zur Behandlung von Lipidstoffwechselstörungen.

8. Verfahren zur Herstellung eines Arzneimittels enthaltend eine oder mehrere der Verbindungen gemäß Anspruch 1, **dadurch gekennzeichnet, daß** der Wirkstoff mit einem pharmazeutisch geeigneten Träger vermischt wird und diese Mischung in eine für die Verabreichung geeignete Form gebracht wird.

9. Verwendung der Verbindungen gemäß Anspruch 1 zur Herstellung eines Medikaments zur Behandlung von Hyperlipidämie.

10. Verwendung der Verbindungen gemäß Anspruch 1 zur Herstellung eines Medikaments zur Senkung des Serumcholesterinspiegels.

11. Verwendung der Verbindungen gemäß Anspruch 1 zur Herstellung eines Medikaments zur Behandlung arteriosklerotischer Erscheinungen.

12. Verwendung der Verbindungen gemäß Anspruch 1 zur Herstellung eines Medikaments zur Behandlung von Insulin Resistenz.

## Claims

1. A compound of the formula I, 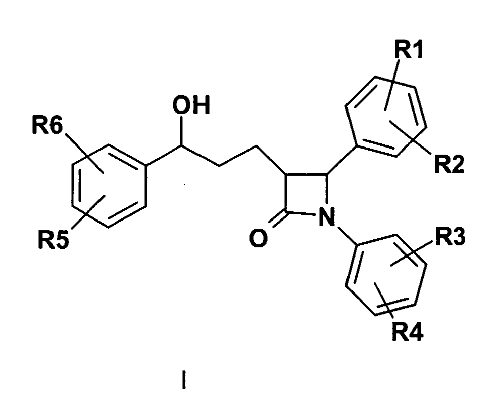 in which
R1, R3, independently of one another are -(CH₂)₀₋₁-NH-(C=O)₀₋₁-(C₀-C₂₅)-alkylene-(C=O)₀₋₁-N(R7)₀₋₁-LAG, where one or more carbon atoms of the alkylene radical may be replaced by oxygen atoms,
H, F, Cl, Br, I, CF₃, NO₂, CN, COOH, COO(C₁-C₆)-alkyl, CONH₂, CONH(C₁-C₆)-alkyl, CON[(C₁-C₆)-alkyl]₂, (C₁-C₆)-alkyl, (C₂-C₆)-alkenyl, (C₂-C₆)-alkynyl, O-(C₁-C₆)-alkyl, where one, more or all hydrogens in the alkyl radicals may be replaced by fluorine;
SO₂-NH₂, SO₂NH(C₁-C₆)-alkyl, SO₂N[(C₁-C₆)-alkyl]₂, S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n = 0 - 6 and the phenyl radical may be substituted up to two times by F, Cl, Br, OH, CF₃, NO₂, CN, OCF₃, O-(C₁-C₆)-alkyl, (C₁-C₆)-alkyl, NH₂;
NH₂, NH-(C₁-C₆)-alkyl, N((C₁-C₆)-alkyl)₂, NH(C₁-C₇)-acyl, phenyl, O-(CH₂)ₙ-phenyl, where n = 0 - 6, where the phenyl ring may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
R2, R4, R5, R6 independently of one another are -(CH₂)₀₋₁-NH-(C=O)₀₋₁-(C₃-C₂₅)-alkylene-(C=O)₀₋₁-N(R₇)₀₋₁-LAG,where one or more carbon atoms of the alkylene radical may be replaced by oxygen atoms,
H, F, Cl, Br, I, CF₃, NO₂, CN, COOH, COO(C₁-C₆)-alkyl, CONH₂, CONH(C₁-C₆)-alkyl, CON[(C₁-C₆)-alkyl]₂, (C₁-C₆)-alkyl, (C₂-C₆)-alkenyl, (C₂-C₆)-alkynyl, O-(C₁-C₆)-alkyl, where one, more or all hydrogens in the alkyl radicals may be replaced by fluorine;
SO₂-NH₂, SO₂NH(C₁-C₆)-alkyl, SO₂N[(C₁-C₆)-alkyl]₂, S-(C₁-C₆)-alkyl, S-(CH₂)ₙ-phenyl, SO-(C₁-C₆)-alkyl, SO-(CH₂)ₙ-phenyl, SO₂-(C₁-C₆)-alkyl, SO₂-(CH₂)ₙ-phenyl, where n = 0 - 6 and the phenyl radical may be substituted up to two times by F, Cl, Br, OH, CF₃, NO₂, CN, OCF₃, O-(C₁-C₆)-alkyl, (C₁-C₆)-alkyl, NH₂;
NH₂, NH-(C₁-C₆)-alkyl, N((C₁-C₆)-alkyl)2, NH(C₁-C₇)-acyl, phenyl, O-(CH₂)ₙ-phenyl, where n = 0 - 6 and the phenyl ring may be mono- to trisubstituted by F, Cl, Br, I, OH, CF₃, NO₂, CN, OCF₃, O-(C₁-C₆)-alkyl, (C₁-C₆)-alkyl, NH₂, NH(C₁-C₆)-alkyl, N((C₁-C₆)-alkyl)₂, SO₂-CH₃, COOH, COO-(C₁-C₆)-alkyl, CONH₂;
R7 is H, CH₃;
(LAG) is a sugar residue;
where one of the radicals R1 or R3 has the meaning -(CH₂)₀₋₁-NH-(C=O)₀₋₁-(C₀-C₂₅)-alkylene-(C=0)₀₋₁-N(R7)₀₋₁-LAG,where one or more carbon atoms of the alkylene radical may be replaced by oxygen atoms, and its pharmaceutically acceptable salts.

2. A medicament comprising one or more of the compounds as claimed in claim 1.

3. A medicament comprising one or more of the compounds as claimed in claim 1 and at least one further active compound.

4. The medicament as claimed in claim 3, comprising, as further active compound, one or more compounds which normalize lipid metabolism.

5. The medicament as claimed in claim 3 or 4, comprising, as further active compound, one or more antidiabetics, hypoglycemically active compounds, HMGCoA reductase inhibitors, cholesterol absorption inhibitors, PPAR gamma agonists, PPAR alpha agonists, PPAR alpha/gamma agonists, fibrates, MTP inhibitors, bile acid absorption inhibitors, CETP inhibitors, polymeric bile acid adsorbers, LDL receptor inducers, ACAT inhibitors, antioxidants, lipoprotein lipase inhibitors, ATP citrate lyase inhibitors, squalene synthetase inhibitors, lipoprotein(a) antagonists, lipase inhibitors, insulins, sulfonyl ureas, biguanides, meglitinides, thiazolidindiones, α-glucosidase inhibitors, active compounds which act on the ATP-dependent potassium channel of the beta cells, CART agonists, NPY agonists, MC4 agonists, orexin agonists, H3 agonists, TNF agonists, CRF agonists, CRF BP antagonists, urocortin agonists, β3 agonists, MSH (melanocyt-stimulating hormone) agonists, CCK agonists, serotonin-reuptake inhibitors, mixed serotonin and noradrenergic compounds, 5HT agonists, bombesin agonists, galanin antagonists, growth hormones, growth hormone-releasing compounds, TRH agonists, decoupling protein 2- or 3-modulators, leptin agonists, DA agonists (bromocriptine, doprexin), lipase/amylase inhibitors, PPAR modulators, RXR modulators or TR-β-agonists or amphetamines.

6. The medicament as claimed in claim 5, comprising, as HMGCoA reductase inhibitor, simvastatin, fluvastatin, pravastatin, lovastatin, atorvastatin, cerivastatin or rosuvastatin.

7. A compound as claimed in claim 1 for use as a medicament for the treatment of impaired lipid metabolism.

8. A process for preparing a medicament comprising one or more of the compounds as claimed in claim 1, which comprises mixing the active compound with a pharmaceutically acceptable carrier and bringing this mixture into a form suitable for administration.

9. The use of the compounds as claimed in claim 1 for preparing a medicament for treating hyperlipidemia.

10. The use of the compounds as claimed in claim 1 for preparing a medicament for lowering the serum cholesterol concentration.

11. The use of the compounds as claimed in claim 1 for preparing a medicament for treating arteriosclerotic manifestations.

12. The use of the compounds as claimed in claim 1 for treating insulin resistance.

## Revendications

1. Composés de formule I, 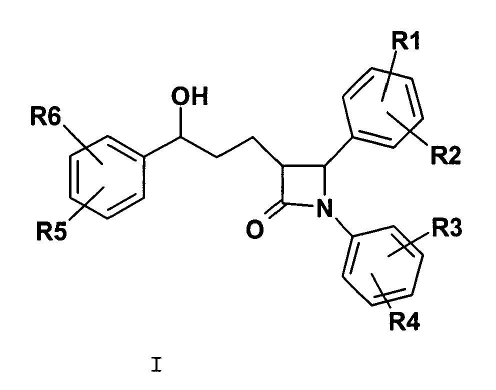 dans laquelle
R1, R3, indépendamment l'un de l'autre, représentent un groupe - (CH₂)₀₋₁-NH- (C=O)₀₋₁-(C₀-C₂₅) alkylène- (C=O)₀₋₁-N(R7)₀₋₁-LAG, dans lequel un ou plusieurs atomes de carbone du radical alkylène peuvent être remplacés par des atomes d'oxygène ;
un atome d'hydrogène, un atome de fluore, un atome de chlore, un atome de brome, un atome d'iode, un groupe CF₃, un groupe NO₂, un groupe CN, un groupe COOH, un groupe COO(C₁-C₆) alkyle, un groupe CONH₂, un groupe CONH(C₁-C₆) alkyle, un groupe CON[(C₁-C₆) alkyle]₂, un groupe alkyle en (C₁-C₆), un groupe alcényle en (C₂-C₆), un groupe alcynyle en (C₂-C₆), un groupe O-(C₁-C₆)alkyle, où dans les radicaux alkyle, un, plusieurs, ou tous les atomes d'hydrogène peuvent être remplacés par un atome de fluore ;
un groupe SO₂-NH₂, un groupe SO₂NH(C₁-C₆)alkyle, un groupe SO₂N[(C₁-C₆)alkyle]₂, un groupe S-(C₁-C₆)alkyle, un groupe S-(CH₂)ₙ-phényle, un groupe SO-(C₁-C₆)alkyle, un groupe SO-(CH₂)ₙ-phényle, un groupe SO₂-(C₁-C₆) alkyle, un groupe SO₂- (CH₂)ₙ-phényle, dans lequel n peut valoir de 0 à 6 et le radical phényle peut être substitué jusqu'à deux fois par un atome de fluore, un atome de chlore, un atome de brome, un groupe OH, un groupe CF₃, un groupe NO₂, un groupe CN, un groupe OCF₃, un groupe O-(C₁-C₆)alkyle, un groupe alkyle en (C₁-C₆), un groupe NH₂ ;
un groupe NH₂, un groupe NH-(C₁-C₆)alkyle, un groupe N((C₁-C₆)alkyle)₂, un groupe NH(C₁-C₇)acyle, un groupe phényle, un groupe O-(CH₂)ₙ-phényle, dans lequel n peut valoir de 0 à 6, où le noyau phényle peut être substitué de une à trois fois par un atome de fluore, un atome de chlore, un atome de brome, un atome d'iode, un groupe OH, un groupe CF₃, un groupe NO₂, un groupe CN, un groupe OCF₃, un groupe O-(C₁-C₆)alkyle, un groupe (C₁-C₆)alkyle, un groupe NH₂, un groupe NH(C₁-C₆)alkyle, un groupe N((C₁-C₆)alkyle)₂, un groupe SO₂-CH₃, un groupe COOH, un groupe COO-(C₁-C₆)alkyle, un groupe CONH₂ ;
R2, R4, R5, R6, indépendamment les uns des autres,
représentent un groupe - (CH₂)₀₋₁-NH-(C=O)₀₋₁-(C₃-C₂₅)alkylène-(C=O)₀₋₁-N(R7)₀₋₁-LAG, dans lequel un ou plusieurs atomes de carbone du radical alkylène peuvent être remplacés par des atomes d'oxygène ;
un atome d'hydrogène, un atome de fluore, un atome de chlore, un atome de brome, un atome d'iode, un groupe CF₃, un groupe NO₂, un groupe CN, un groupe COOH, un groupe COO(C₁-C₆) alkyle, un groupe CONH₂, un groupe CONH (C₁-C₆) alkyle, un groupe CON[(C₁-C₆) alkyle]₂, un groupe alkyle en (C₁-C₆), un groupe alcényle en (C₂-C₆), un groupe alcynyle en (C₂-C₆), un groupe O-(C₁-C₆)alkyle, où dans les radicaux alkyle, un, plusieurs, ou tous les atomes d'hydrogène peuvent être remplacés par un atome de fluore ;
un groupe SO₂-NH₂, un groupe SO₂NH(C₁-C₆)alkyle, un groupe SO₂N[(C₁-C₆)alkyle]₂, un groupe S-(C₁-C₆)alkyle, un groupe S-(CH₂)ₙ-phényle, un groupe SO-(C₁-C₆)alkyle, un groupe SO-(CH₂)ₙ-phényle, un groupe SO₂-(C₁-C₆) alkyle, un groupe SO₂-(CH₂)ₙ-phényle, dans lequel n peut valoir de 0 à 6 et le radical phényle peut être substitué jusqu'à deux fois par un atome de fluore, un atome de chlore, un atome de brome, un groupe OH, un groupe CF₃, un groupe NO₂, un groupe CN, un groupe OCF₃, un groupe O-(C₁-C₆)alkyle, un groupe alkyle en (C₁-C₆), un groupe NH₂ ;
un groupe NH₂, un groupe NH-(C₁-C₆)alkyle, un groupe N((C₁-C₆)alkyle)₂, un groupe NH(C₁-C₇)acyle, un groupe phényle, un groupe O-(CH₂)ₙ-phényle, dans lequel n peut valoir de 0 à 6, où le noyau phényle peut être substitué de une à trois fois par un atome de fluore, un atome de chlore, un atome de brome, un atome d'iode, un groupe OH, un groupe CF₃, un groupe NO₂, un groupe CN, un groupe OCF₃, un groupe O-(C₁-C₆)alkyle, un groupe (C₁-C₆)alkyle, un groupe NH₂, un groupe NH(C₁-C₆)alkyle, un groupe N((C₁-C₆)alkyle)₂, un groupe SO₂-CH₃, un groupe COOH, un groupe COO-(C₁-C₆)alkyle, un groupe CONH₂ ;
R7 représente un atome d'hydrogène, un groupe CH₃ ;
(LAG) représente un radical glucidique ;
où l'un des radicaux R1 à R3 représente un groupe - (CH₂)₀₋₁-NH- (C=O)₀₋₁- (C₀-C₂₅) alkylène- (C=O)₀₋₁-N(R7)₀₋₁-LAG, dans lequel un ou plusieurs atomes de carbone du radical alkylène radical peuvent être remplacé par des atomes d'oxygène,
ainsi que leurs sels pharmaceutiquement acceptables.

2. Médicament comprenant un ou plusieurs des composés selon la revendication 1.

3. Médicament comprenant un ou plusieurs des composés selon la revendication 1 et au moins un ingrédient actif supplémentaire.

4. Médicament selon la revendication 3, **caractérisé en ce qu'**il comprend, en tant qu'ingrédient actif supplémentaire, un ou plusieurs composés qui normalisent le métabolisme lipidique.

5. Médicament selon la revendication 3 ou 4, **caractérisé en ce qu'**il comprend, en tant qu'ingrédient actif supplémentaire, un ou plusieurs agents antidiabétiques, ingrédients actifs hypoglycémiants, inhibiteurs de HMGCoA-réductase, inhibiteurs de la résorption du cholestérol, agonistes gamma de PPAR, agonistes alpha de PPAR, agonistes alpha/gamma de PPAR, fibrates, inhibiteurs de MTP, inhibiteurs de la résorption de l'acide biliaire, inhibiteurs de CETP, agents adsorbants polymère de l'acide biliaire, inducteurs de récepteur de LDL, inhibiteurs d'ACAT, agents antioxydants, inhibiteurs de lipoprotéine-lipase, inhibiteurs d'ATP-citrate-lyase, inhibiteurs de squalène synthétase, antagonistes de lipoprotéine(a), inhibiteurs de lipase, insulines, sulfonylurées, biguanides, méglitinides, thiazolidinediones, inhibiteurs de α-glucosidase, ingrédients actifs agissant sur le canal potassique ATP-dépendent des cellules bêta, agonistes de CART, agonistes de NPY, agonistes de MC4, agonistes de l'orexine, agonistes H3, agonistes de TNF, agonistes de CRF, antagonistes de CRF BP, antagonistes de l'urocortine, agonistes β3, agonistes de la MSH (hormone mélano-stimulante), agonistes de CCK, inhibiteurs du recaptage de la sérotonine, composés mixtes sérotoninérgiques et noradrénergiques, agonistes 5HT, agonistes de la bombésine, antagonistes de la galanine, hormones de croissance, composés libérant une hormone de croissance, agonistes de TRH, modulateurs de la protéine découplante 2 ou 3, agonistes de la leptine, agonistes de DA (bromocriptine, doprexine), inhibiteurs de lipase/amylase, modulateurs de PPAR, modulateurs de RXR ou agonistes TR-β ou amphétamines.

6. Médicament selon la revendication 5, **caractérisé en ce qu'**il comprend, en tant qu'inhibiteur de HMGCoA-réductase, de la simvastatine, de la fluvastatine, de la pravastatine, de la lovastatine, de l'atorvastatine, de la cerivastatine ou de la rosuvastatine.

7. Composés selon la revendication 1 pour une application en tant que médicament destiné au traitement de troubles du métabolisme lipidique.

8. Procédé de préparation d'un médicament comprenant un ou plusieurs des composés selon la revendication 1, **caractérisé en ce que** l'ingrédient actif est mélangé avec un support pharmaceutiquement approprié et ce mélange est mis sous une forme appropriée pour l'administration.

9. Utilisation des composés selon la revendication 1 pour la préparation d'un médicament destiné au traitement de l'hyperlipidémie.

10. Utilisation des composés selon la revendication 1 pour la préparation d'un médicament destiné à réduire le taux de cholestérol sérique.

11. Utilisation des composés selon la revendication 1 pour la préparation d'un médicament destiné au traitement de manifestations artériosclérotiques.

12. Utilisation des composés selon la revendication 1 pour la préparation d'un médicament destiné au traitement de la résistance à l'insuline.
